# EUROPEAN PATENT SPECIFICATION

(11) **EP 1 409 498 B1**
(45) Date of publication and mention of the grant of the patent: **26.03.2008**
(21) Application number: 01928115.3
(22) Date of filing: 11.05.2001
(51) Int. Cl.: C07H 3/04, C07H 15/207, C07H 3/06, A61K 31/70

(54) **INOSITOL PHOSPHOGLYCAN DERIVATIVES AND THEIR MEDICAL USES**
INOSITOLPHOSPHOGLYCANDERIVATE UND IHRE MEDIZINISCHE VERWENDUNG
DERIVES DE PHOSPHOGLYCANE D'INOSITOL ET LEURS UTILISATIONS EN MEDECINE

(30) Priority: 12.05.2000 GB 0011593; 12.05.2000 US 203598 P; 02.03.2001 US 798004
(43) Date of publication of application: 21.04.2004
(73) Proprietor: Rodaris Pharmaceuticals Limited, Oxford, OX4 4GA (GB)
(72) Inventor: RADEMACHER, Thomas, William, Oxfordshire OX1 5EY (GB); CARO, Hugo, Norberto, Barcelona 08036 (ES); FRANCOIS, Irene Florey House, Horsell Woking, Surrey,GU21 4BE (GB); MARTIN-LOMAS, Manuel, E-41013 Seville (ES)
(74) Representative: Kiddle, Simon John
(86) International application number: PCT/GB2001/002098
(87) International publication number: WO 2001/085745

(56) References cited:
- EP-A- 0 845 475
- WO-A-00/15254
- WO-A-98/10791
- WO-A-98/50049
- WO-A-99/38516
- PATENT ABSTRACTS OF JAPAN vol. 1995, no. 01, 28 February 1995 (1995-02-28) & JP 06 293790 A (KYOWA HAKKO KOGYO CO LTD), 21 October 1994 (1994-10-21)
- PATENT ABSTRACTS OF JAPAN vol. 016, no. 375 (C-0973), 12 August 1992 (1992-08-12) & JP 04 120089 A (RIKAGAKU KENKYUSHO;OTHERS: 01), 21 April 1992 (1992-04-21)
- PATENT ABSTRACTS OF JAPAN vol. 016, no. 020 (C-0902), 20 January 1992 (1992-01-20) & JP 03 237101 A (RIKAGAKU KENKYUSHO;OTHERS: 01), 23 October 1991 (1991-10-23)
- PATENT ABSTRACTS OF JAPAN vol. 012, no. 479 (C-552), 14 December 1988 (1988-12-14) & JP 63 196596 A (KURITA WATER IND LTD;OTHERS: 01), 15 August 1988 (1988-08-15)
- MÜLLER, G. ET AL.: "Structure-activity relationship of synthetic phosphoinositolglycans mimicking metabolic insulin action" BIOCHEMISTRY, vol. 37, no. 38, 1998, pages 13421-13436, XP002135715
- JAWOREK C H ET AL: "Synthesis of an Inositol-Containing Trisaccharide Related to Insulin Signal Transduction" TETRAHEDRON LETTERS, ELSEVIER SCIENCE PUBLISHERS, AMSTERDAM, NL, vol. 40, no. 4, 22 January 1999 (1999-01-22), pages 667-670, XP004151413 ISSN: 0040-4039
- DEEG, M.A. ET AL.: "Inositol glycan phosphate derived from human erythrocyte acetylcholinesterase glycolipid anchor and inositol cyclic 1,2-phosphate antagonize glucagon activation of glycogen phosphorylase" DIABETES, vol. 42, no. 9, 1993, pages 1318-23, XP001018200
- FERGUSON, A.J. ET AL: "Molecular species analysis and quantification of the glycosylphosphatidylinositol intermediate glycolipid C from Trypanosoma brucei" MOL. BIOCHEM. PARASITOL., vol. 77, no. 2, 1996, pages 137-45, XP001017844

## Description

### Field of the Invention

The present invention relates to compounds and their uses, and in particular to compounds which have a mimetic or antagonistic property of an inositol phosphoglycan or a free GPI precursor of an IPG, and the uses of these compounds, e.g. to treat a condition ameliorated by administration of an IPG second messenger or an IPG antagonist thereof.

### Background of the Invention

Many of the actions of growth factors and hormones on cells are thought to be mediated by a family of inositol phosphoglycan (IPG) second messengers ^{[13]}. It is thought that the source of IPGs is a "free" form of glycosyl phosphatidylinositol (GPI) situated in cell membranes. IPGs are thought to be released by the action of phosphatidylinositol-specific phospholipases following binding of growth factors to receptors on the cell surface. There is evidence that IPGs mediate the action of a large number of growth factors including insulin, nerve growth factor, hepatocyte growth factor, insulin-like growth factor I (IGF-I), fibroblast growth factor, transforming growth factor β, the action of IL-2 on B-cells and T-cells, ACTH signalling of adrenocortical cells, IgE, FSH and hCG stimulation of granulosa cells, thyrotropin stimulation of thyroid cells, cell proliferation in the early developing ear and rat mammary gland.

Partially characterised inositolphosphoglycans (IPGs) have been postulated to mediate the action of a number of growth factors and hormones including insulin and insulin-like growth factor I (IGF-I) ^{[1]}. Despite their isolation from several tissues type, the precise chemical structures of these IPGs are, however, still unknown and two main structural groups have been proposed on the basis of the chemical composition ^{[2,3]} which display different biological activity and tissue distribution ^{[4]}; the family of glucosamine-*myo*-inositol containing IPGs (IPG-A) and the family of *chiro*-inositolgalactosamine containing IPGs (IPG-P).

In an attempt to establish the minimal structural requirements for biological activity, a number of compounds containing some of the basic structural motifs that have been postulated for IPG mediators have been synthesised in the art ^{[5]}. These synthetic compounds include *O*-(2-amino-2-deoxy-D-glucopyranosyl)-α(1-6)-*chiro*-inositol 1-phosphate and *O*-(2-amino-2-deoxy-D-glucopyranosyl)-a(1-6)-*myo*-inositol 1-phosphate ^{[6]}.

US Patent No: 6,004,938 (Hoechst) discloses a group of synthetic inositol glycans having insulin-like action. The compounds are based on 2-6 monsaccharide units linked to an inositol moiety. The examples in the patent all employ *myo*-inositol and are composed of 5 or 6 units apart from two pseudo-trisaccharide compounds G and H. Compounds G and H are HO-PO(H)O-6Man-α(1-4)-GluN-α(1-6)-(L)inositol-1.2(cyclic) phosphate and HO-PO(H)O-6Man-α(1-4)-GluN-α(1-6)-(L)inositol, otherwise known as *O*-(6-hydrogenphosphonate-α-D-mannopyranosyl)-(1-4)-(2-ammonio-2-deoxy-α-D-glucopyranosyl)-(1-6)-L-*myo*-inositol-1 ,2-cyclic phosphate and *O*-(6-hydrogenphosphonate-α-D-mannopyranosyl)-(1-4)-(2-amino-2-deoxy-α-D-glucopyranosyl)-L-*myo*-inositol. The properties of exemplified compounds are investigated in lipogenesis and glucose transport assays employing rat fat cells.

WO96/14075 (University of Virginia) discloses a generic family of compounds D-hexosamines linked to an inositol via a β1,4-linkage. The inositols can be*myo* or chiro-inositol or pinitol, while the hexosamines are glucosamine or galactosamine. However, this application describes the synthesis of just two compounds 4-*O*-(2-deoxy-2-amino-β-D-galactopyranosyl)-D-pinitol and 4-*O*-(2-deoxy-2-ammo-β-D-galactopyranosyl)-D-chiro-inositol, or in IUPAC notation *O*-(2-amino-2-deoxy-β-D-galactopyranosyl)-(1-4)-D-pinitol and *O*-(2-amino-2-deoxy-β-D-galactopyranosyl)-(1-4)-D-chiro-inositol.

WO99/06421 (University of Virginia) describes synthetic insulin mimetic substances and includes a general formula I showing β1,4-linked disaccharides. However, despite this the compounds synthesised in this application are exactly the same as those disclosed in the applicant's earlier application, WO96/14075.

A multi-step synthesis of a IPG-P mimetic from glucose has been previously reported in Jaramillo et al ^{[6]}, which discloses a compound called C4, 1-D-6-*O*-(2-amino-2-deoxy-α-D-glucopyranosyl)-*chiro*-inositol 1-phosphate. A further synthesis of C4 is described in our co-pending International Patent Application PCT/GB99/03715 (Rademacher Group Limited). Zapata et al ^{[16]} discloses three other compounds C1-C3 which are:
- C1: 1-D-4-*O*-(2-amino-2-deoxy-α-D-glucopyranosyl)-*myo*-inositol 1-phosphate.
- C2: 1-D-6-*O*-(2-amino-2-deoxy-α-D-glucopyranosyl)-*myo*-inositol 1-phosphate.
- C3: 1-D-6-*O*-(2-amino-2-deoxy-α-D-glucopyranosyl)-myo-inositol 1,2 cyclicphosphate.

It remains a significant problem in the art to produce synthetic compounds which can mimic one or more of the activities of inositol phosphoglycans or which act as antagonists of IPGs.

### Summary of the Invention

Broadly, the present invention relates to IPG mimetic and antagonist compounds and to methods of producing the compounds and to their medical uses. The compounds disclosed herein are useful as synthetic mimetics of IPG-P or IPG-A second messengers and/or growth factors whose action is mediated by IPGs, as synthetic mimetics of free GPI precursors or IPGs, or as competitive antagonists of IPGs. In particular, the compounds are based on the 1,6 linkage of two or more sugar residues to a chiro-inositol.

Accordingly, in a first aspect, the present invention provides a compound represented by the general formula:

Y-X-1,6-chiro-inositol

wherein:
X represents a sugar radical;
Y represents one to three sugar radicals;
the sugar residue is unsubstituted or substituted with between one and four groups, and the cyclitol is unsubstituted or is further substituted with between one and four groups, the group or groups being independently selected from:
   (a) a phosphoryl group which is phosphate -O-P(O)(OH)₂; thiophosphate -O-P(S)(OH)₂; phosphate esters -O-P(O)(OR)₂; thiophosphate esters -O-P(S)(OR)₂; phosphonate -OP(O)OHR; thiophosphonate -O-P(S)OHR; substituted phosphonate -O-P(O)OR₁R₂; substituted thiophosphonate -O-P(S)OR₁R₂; -O-P(S)(OH)(SH); or cyclic phosphate;
   (b) a phosphorus containing compound which is phosphoramidite -O-P(OR)-NR₁R₂ or phosphoramidate -O-P(O)(OR)-NR₁R₂;
   (c) a sulphur group which is -O-S(O)(OH), -SH, -SR, -S(→O)-R, -S(O)₂R, RO-S(O)₂,-O-SO₂NH₂, -O-SO₂R₁R₂ or sulphamide -NHSO₂NH₂;
   (d) an amino group which is -NHR, -NR₁R₂, -NHAc, -NHCOR, -NH-O-COR, -NHSO₃,-NHSO₂R or -N(SO-)R)₂, and/or an amidino group which is -NH-C(=NH)NH₂ and/or an ureido group which is -NH-CO-NR₁R₂ or a thiouriedo group which is -NH-C(S)-NH₂;
   (e) a hydroxy group or substituted hydroxy groups which is -OR₃, where R₃ is C₁₋₁₀ unsubstituted or substituted alkyl, e.g. CHF₂ or CF₃, alkoxyalkyl, aryloxyalkyl, cycloalkyl, alkenyl (unsubstituted alkyl), alkylene (C₃₋₇ cycloalkyl), -OCOR, aryl, heteroaryl, acetal, or where two hydroxyl groups are joined as a ketal;
   (f) a halogen substituent which is fluorine or chlorine;
   (g) a hydrogen to provide a deoxy sugar;
wherein R, R₁ and R₂ are independently hydrogen or C₁₋₁₀ unsubstituted or substituted alkyl or aryl;
or a salt, coordination complex with metal ions, ester, free acid or base, or hydrate therof,
or a glycolipid derivative in which a lipid moiety cleavable by a phospholipase enzyme is a substituent of a sugar residue or the cyclitol.

The compounds may be provided as racemic or diasteromeric mixtures, resolved or partially resolved optical isomers, and as pharmaceutically acceptable salts, esters and derivatives as discussed in more detail below.

Examples of compounds within this embodiment of the invention are RGL1021, RGL1022, and compounds 19 and 25.

Preferably, the X or Y sugar residue is a hexose or a pentose, and may be an aldose or a ketose. The sugar residue can be a member of the D or L series and can include amino sugars, deoxy sugars and their uronic acid derivatives. Preferably, where the sugar residue is a hexose, it is selected from the group consisting of glucose, galactose or mannose, or substituted hexose sugar residues such as an amino sugar residue such as hexosamine, galactosamine or glucosamine, and more preferably D-glucosamine (2-amino-2-deoxy-D-glucose) or D-galactosamine (2-amino-2-deoxy-D-galactose). Preferred pentose sugar residues include arabinose, fucose and ribose. The X or Y sugar residue is optionally substituted at one, two, three or four positions, other than the anomeric position or the position of linkage of the other radical or to the chiro-inositol.

The chiro-inositol may be in its D or L form, and is optionally substituted at one or more of the positions other than the position of linkage to the sugar radical. The sugar radical is optionally substituted at one, two, three or four positions other than at the position of linkage to the inositol moiety (the anomeric position). Examples of substituted groups include CF₃, X(CH₂)ₙ-O- (where X is hydrogen, or substituted or unsubstituted alkyl), CHF₂O-. In further embodiments, the chro-inositol may have one or more of the hydroxyl groups through which the substituents described above are removed so that any substituent(s) are linked to the ring carbon atom. The sugar residue is optionally substituted at one, two, three, or four positions other than at the position of linkage to the inositol moiety.

Preferably the X and Y sugar residues are linked to each other via a 1,1 linkage, 1,2 linkage, 1,3 linkage, 1, 4 linkage or 1,6 linkage. The linkage between the units may be an a or β linkage. The linkage of the X sugar residue to the chiro-inositol is a 1,6 linkage via one of the oxygen atoms of the chiro-inositol moiety. However, this oxygen atom can be replaced one or more times by -CH₂- or -S- groups.

To avoid confusion, the numbering system used herein is clarified with reference to the following structures. Importantly, as the *chiro*-inositol molecule contains a C2 plane of symmetry and positions I and 6 are equivalents. Therefore, for instance, compounds containing a β(1,6) linkage can be regarded as β(1,1) if there are no other substituents to define priority in the numbering system. Monosaccharide residues and oligosaccharides are named and numbered according to the recommendation proposed by the Joint Commission on Biochemical Nomenclature, International Union of Pure and Applied Chemistry and International Union of Biochemistry and Molecular Biology.

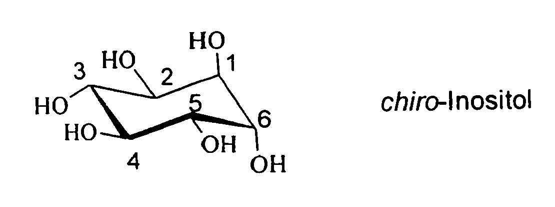

In preferred embodiments, the present invention provides a compound, or a substituted form thereof as defined above, selected from the group consisting of:
- RGL1021: *O*-(D-galactopyranosyl)-α(1,4)-*O*-(2'-amino-2'-deoxy-D-glucanopyranosyl)-α(1,6)-*chiro*-inositol.
- RGL1022: *O*-(D-galactopyranosyl)-α(1,4)-*O*-(2'-amino-2'-deoxy-D-glucanopyranosyl)-α(1,6)-*chiro*-inositol-1-phosphate.
- Compound 25: *O*-α-D-Mannopyranosyl-(1-2)-*O*-α-D-mannopyranosyl-(1-6)-*O*-α-D-mannopyranosyl-(1-4)-*O*-2 ammonio-2-deoxy-α-D-glucopyranosyl-(1-6)-D-*chiro*-inositol-1-phosphate.
- Compound 19: *O*-β-D-galactopyranosyl-(1-4)-2-ammonio-2-deoxy-α-D-galactopyranosyl-(1-6)-D-*chiro*-inositol-1-phosphate.

In a further aspect, the present invention provides methods for making the compounds of the invention or their intermediates as set out in the following experimental description and the schemes. In a further related aspect, the present invention further relates to compounds which are the novel intermediates described herein.

In a further aspect, the present invention provides one or more of the above compounds for use in a method of medical treatment. The compounds may be useful as IPG mimetics or IPG antagonists, e.g. as competitive antagonists.

In a further aspect, the present invention provides the use of one or more of the above compounds for the preparation of a medicament for the treatment of a condition ameliorated by the administration of an inositol phosphoglycan (IPG) second messenger or an IPG antagonist. Examples of such conditions are set out in the pharmaceutical uses section below.

Embodiments of the invention will now be described by way of example and not limitation with reference to the accompanying drawings.

### Brief Description of the Figures

Scheme 1 shows the synthesis of compound 4.
Scheme 2 shows the synthesis of RGL1021 from compound 4.
Scheme 3 shows the production of RGL1022 from compound 7.
Scheme 4 shows the production of compound 19, a derivative of compound 4.
Schemes 5(I) and 5(II) show the synthesis of compound **25.**

### Detailed Description

### Inositol phosphoglycans (IPGs)

IPG-A mediators modulate the activity of a number of insulin-dependent enzymes such as cAMP dependent protein kinase (inhibits), adenylate cyclase (inhibits) and cAMP phospho-diesterases (stimulates). In contrast, IPG-P mediators modulate the activity of insulin-dependent enzymes such as pyruvate dehydrogenase phosphatase (stimulates) and glycogen synthase phosphatase (stimulates). The A-type mediators mimic the lipogenic activity of insulin on adipocytes, whereas the P-type mediators mimic the glycogenic activity of insulin on muscle. Both A-and P-type mediators are mitogenic when added to fibroblasts in serum free media. The ability of the mediators to stimulate fibroblast proliferation is enhanced if the cells are transfected with the EGF-receptor. A-type mediators can stimulate cell proliferation in the chick cochleovestibular ganglia.

Soluble IPG fractions having A-type and P-type activity have been obtained from a variety of animal tissues including rat tissues (liver, kidney, muscle, brain, adipose, heart) and bovine liver. IPG-A and IPG-P biological activity has also been detected in human liver and placenta, malaria parasitized RBC and mycobacteria. The ability of an anti-inositolglycan antibody to inhibit insulin action on human placental cytotrophoblasts and BC3H1 myocytes or bovine-derived IPG action on rat diaphragm and chick cochleovestibular ganglia suggests cross-species conservation of many structural features. However, it is important to note that although the prior art includes these reports of IPG-A and IPG-P activity in some biological fractions, the purification or characterisation of the agents responsible for the activity is not disclosed.

IPG-A substances are cyclitol-containing carbohydrates, also containing Zn²⁺ ions and phosphate and having the properties of regulating lipogenic activity and inhibiting cAMP dependent protein kinase. They may also inhibit adenylate cyclase, be mitogenic when added to EGF-transfected fibroblasts in serum free medium, and stimulate lipogenesis in adipocytes.

IPG-P substances are cyclitol-containing carbohydrates, also containing Mn²⁺ and/or Zn²⁺ ions and phosphate and having the properties of regulating glycogen metabolism and activating pyruvate dehydrogenase phosphatase. They may also stimulate the activity of glycogen synthase phosphatase, be mitogenic when added to fibroblasts in serum free medium, and stimulate pyruvate dehydrogenase phosphatase.

Methods for obtaining A-type and P-type mediators are set out in Caro et al, 1997, and in WO98/11116 and WO98/11117. Protocols for determining characteristic IPG biological activities such as PDH activation, PKA inhibition, acetylCoA activation, fructose-1,6-bis-phosphatase activity and lipogenesis are well known in the art, e.g. as described in Caro et al ^{[14]}.

### Drug Formulation

The compounds of the invention may be derivatised in various ways. As used herein "derivatives" of the compounds includes salts, coordination complexes with metal ions such as Mn²⁺ and Zn²⁺, esters such as *in vivo* hydrolysable esters, free acids or bases, hydrates or glycolipid derivatives.

Salts of the compounds of the invention are preferably physiologically well tolerated and non toxic. Many examples of salts are known to those skilled in the art. Compounds having acidic groups, such as phosphates or sulfates, can form salts with alkaline or alkaline earth metals such as Na, K, Mg and Ca, and with organic amines such as triethylamine and Tris (2-hydroxyethyl)amine. Salts can be formed between compounds with basic groups, e.g. amines, with inorganic acids such as hydrochloric acid, phosphoric acid or sulfuric acid, or organic acids such as acetic acid, citric acid, benzoic acid, fumaric acid, or tartaric acid. Compounds having both acidic and basic groups can form internal salts.

Esters can be formed between hydroxyl or carboxylic acid groups present in the compound and an appropriate carboxylic acid or alcohol reaction partner, using techniques well known in the art.

Some of the above defined derivatives may act as prodrugs of the compounds are convertible *in vivo* or *in vitro* into one of the parent compounds. Typically, at least one of the biological activities of compound will be reduced in the prodrug fonn of the compound, and can be activated by conversion of the prodrug to release the compound or a metabolite of it. An example of prodrugs are glycolipid derivatives in which one or more lipid moieties are provided as substituents on the sugar residue or the chiro-inositol moieties, leading to the release of the free form of the compound by cleavage with a phospholipase enzyme.

### Pharmaceutical Compositions

The compounds described herein or their derivatives can be formulated in pharmaceutical compositions, and administered to patients in a variety of forms, in particular to treat conditions which are ameliorated by the administration of inositol phosphoglycan second messengers or IPG antagonists such as competitive antagonist.

Pharmaceutical compositions for oral administration may be in tablet, capsule, powder or liquid form. A tablet may include a solid carrier such as gelatin or an adjuvant or an inert diluent. Liquid pharmaceutical compositions generally include a liquid carrier such as water, petroleum, animal or vegetable oils, mineral oil or synthetic oil. Physiological saline solution, or glycols such as ethylene glycol, propylene glycol or polyethylene glycol may be included. Such compositions and preparations generally contain at least 0.1 wt% of the compound.

Parental administration includes administration by the following routes: intravenous, cutaneous or subcutaneous, nasal, intramuscular, intraocular, transepithelial, intraperitoneal and topical (including dermal, ocular, rectal, nasal, inhalation and aerosol), and rectal systemic routes. For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, solutions of the compounds or a derivative thereof, e.g. in physiological saline, a dispersion prepared with glycerol, liquid polyethylene glycol or oils.

In addition to one or more of the compounds, optionally in combination with other active ingredient, the compositions can comprise one or more of a pharmaceutically acceptable excipient, carrier, buffer, stabiliser, isotonicizing agent, preservative or anti-oxidant or other materials well known to those skilled in the art. Such materials should be non-toxic and should not interfere with the efficacy of the active ingredient. The precise nature of the carrier or other material may depend on the route of administration, e.g. orally or parentally.

Liquid pharmaceutical compositions are typically formulated to have a pH between about 3.0 and 9.0, more preferably between about 4.5 and 8.5 and still more preferably between about 5.0 and 8.0. The pH of a composition can be maintained by the use of a buffer such as acetate, citrate, phosphate, succinate, Tris or histidine, typically employed in the range from about 1 mM to 50 mM. The pH of compositions can otherwise be adjusted by using physiologically acceptable acids or bases.

Preservatives are generally included in pharmaceutical compositions to retard microbial growth, extending the shelf life of the compositions and allowing multiple use packaging. Examples of preservatives include phenol, meta-cresol, benzyl alcohol, para-hydroxybenzoic acid and its esters, methyl paraben, propyl paraben, benzalconium chloride and benzethonium chloride. Preservatives are typically employed in the range of about 0.1 to 1.0 % (w/v).

Preferably, the pharmaceutically compositions are given to an individual in a "prophylactically effective amount" or a "therapeutically effective amount" (as the case may be, although prophylaxis may be considered therapy), this being sufficient to show benefit to the individual. Typically, this will be to cause a therapeutically useful activity providing benefit to the individual. The actual amount of the compounds administered, and rate and time-course of administration, will depend on the nature and severity of the condition being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980. By way of example, and the compositions are preferably administered to patients in dosages of between about 0.01 and 100mg of active compound per kg of body weight, and more preferably between about 0.5 and 10mg/kg of body weight.

The composition may further comprise one or more other pharmaceutically active agents, either further compounds of the invention, inositol phosphoglycans, growth factors such as insulin, NGF or other growth factors listed below, or other drugs, e.g. those in use for the treatment of diabetes or other conditions set out below.

### Medical Uses

As set out above, IPGs are second messengers for a range of different growth factors and hormones, including insulin, nerve growth factor, hepatocyte growth factor, endothelial growth factor, insulin-like growth factor I (IGF-I), fibroblast growth factor, transforming growth factor β, the action of IL-2 on B-cells and T-cells, ACTH signalling of adrenocortical cells, IgE, FSH and hCG stimulation of granulosa cells, thyrotropin stimulation of thyroid cells, cell proliferation in the early developing ear and rat mammary gland. Consequently, IPGs or their antagonists can be used in the treatment or amelioration of disorders mediated by the growth factors or to mimic specific growth factor or hormone biological activities.

Examples of conditions which can be treated using IPGs or IPG antagonists include, diabetes, obesity, dyslipidaemia, pre-eclampsia, neurotrophic disorders, hepatic damage and adrenal atrophy.

WO98/10791 discloses that pre-eclampsia is characterised by elevated levels of IPG-P and that it can be treated using an IPG-P antagonist. Compounds of the invention which are IPG-P antagonists, e.g. antagonists which compete with wild-type IPG-P but lack one or more of its activities, could be used in the treatment of pre-eclampsia.

The use of both IPG-P and IPG-A and IPG-A antagonists in the diagnosis and treatment of diabetes is disclosed in WO98/11435. This application discloses that in some forms of diabetes the ratio of P:A-type IPGs is imbalanced and can be corrected by administering a medicament containing an appropriate ratio of IPG-P, IPG-A or antagonist(s) thereof. In particular, it describes the treatment of obese type II diabetes (NIDDM) patients with a P-type IPG and/or an A-type IPG antagonist and the treatment of IDDM or lean type II diabetes (body mass index < 27) with a mixture of P- and A-type IPGs, typically in a P:A ratio of about 6:1 for males and 4:1 for females. The compounds and compositions of the present invention can be employed in such types of treatment. More particularly, the compounds are likely to be of use in the treatment of various form of diabetes and diabetic complications including diabetes due to insulin resistance, insulin resistance in type I diabetes and brittle diabetes, obese or lean type II diabetes, and of conditions associated with insulin resistance or insulin underproduction, such as neurotrophic disorders or polycystic ovary syndrome, lipodystrophy, age-related memory loss, and post-ischaemic damage secondary to stroke or post-transplant complications.

The compounds of this invention are also likely to be of use in controlling neuron proliferation or neurite outgrowth, either *in vitro* or *in vivo,* e.g. acting as a nerve or neurite growth factor mimetic second messenger. They may thus have applications in the treatment and/or diagnosis of any condition related to neuron proliferation or neurite differentiation. WO99/38516 discloses that IPG-A and synthetic mimetics thereof cause neuron proliferation, mimicking the action of the growth factor IGF-I. In contrast, IPG-P and synthetic mimetics thereof such as compound C4 cause neurite outgrowth. The neurons may be central (brain and spinal cord) neurons, peripheral (sympathetic, parasympathetic, sensory and enteric) neurons, e.g. the compounds used in the regeneration of peripheral nerves, or motor neurons. Treatments may involve the treatment of damage to nerve, spinal cord or central nervous system damage secondary to trauma, or autoimmune or metabolic damage, or post-ischaemic damage secondary to stroke or post-transplant complications, motor neuron disease, neurodegenerative disorders or neuropathy. Damage to the nervous system includes the results of trauma, stroke, surgery, infection (e.g. by viral agents), ischemia, metabolic disease, toxic agents, or a combination of these or similar causes. Motor neuron disease includes conditions involving spinal muscular atrophy, paralysis or amyotrophic lateral sclerosis. Neurodegenerative disorders include Parkinson's disease, Alzheimer's disease, epilepsy, multiple sclerosis, Huntingdon's chorea and Meniere's disease.

The compounds of the invention may also be useful as hepatocyte growth factor mimetic second messengers, e.g. in the preparation of medicaments for the treatment of hepatic damage caused by infection, alcohol abuse, drug sensitivity, or autoimmunity. The compounds may also be useful as fibroblast growth factor mimetic second messengers or epidermal growth factor mimetic second messengers, e.g. in the preparation of medicaments for the promotion of wound healing following surgery or trauma or tissue damage induced by ischaemia or autoimmunity.

In other embodiments, the compounds of the invention may be useful as adrenal cell growth factor mimetic second messengers or ACTH mimetic second messengers in the preparation of a medicament for the treatment of disease states involving adrenal atrophy.

The compounds of the invention can readily be tested using the assays identified herein to determine their suitability for some or all of the medical uses described above. Thus, even compounds with a relatively low activity in one of the enzymes assays disclosed herein, may be useful by virtue of possessing a different activity, and moreover the pattern of activities can be used to rapidly screen the compounds for suitability in the various medical applications disclosed herein.

| Activity | Diabetes I | Diabetes II | Obesity | Alzheimer's | Neurotrophics |
|---|---|---|---|---|---|
| PDH Kinase | Inhibit | Inhibit | No Effect | Inhibit | No effect |
| PDH Phosphatase | Activate | Activate | No effect | No effect | No effect |
| Acetyl CoA carboxylase I * | Activate | No effect | No effect | No effect | No effect |

| | | | | | |
|---|---|---|---|---|---|
| * found in liver and adipose cytosol. | | | | | |

### Methods of Making the Compounds

Based on the disclosure herein, the knowledge in the art and in references ^{[5-11]}, the skilled person could couple sugar residues and Chiro-inositol together, optionally with one or more substituents.

Useful guidance on the synthesis of the exemplified compounds and for introducing the substituents set out herein is provided by the papers by Gigg & Gigg, Khiar & Martin-Lomas ^{[5]} and Baeschlin et al ^{[18]} and the references cited therein.

Phosphoryl groups such as phosphate, cyclic phosphate or substituted phosphate or cyclic phosphate can be substituted into the compounds of the invention by the phosphate or phosphoramidite method, Bannwath et al, Helvetica Chemica Acta, 70:175-186, 1987 and Yu & Fraser-Reid, Tetrahedron Lett., 29:979-982, 1988.

Phosphate protecting groups can also be synthesized according to the methods disclosed in Hoeben-Weyl, Methods of Organic Chemistry, volume 12/1 or 12/2, Teilheimer, Synthetic Methods of Organic Chemistry, Vol 45. Protecting groups for the OH of sugars include menthoxycarbonyl (MntCO), acetal (in particular, two R groups may together represent a bridging acetal such as O-cyclohexylidene, *O-*isopropylidene or O-benzylidene), *tert*-butyldimethylsilyl (TBDMS), benzyl (Bn), *tert*-butyldiphenylsilyl (TBDPS). Many protecting groups suitable for use in the syntheses and reactions of saccharides are known and are well documented in standard reference works. The choice depends in part on the route by which the compound is synthesised and/or on the uses to which it is to be put, including the reactions which it is subsequently intended to undergo.

### Bioactivity Assays

The compounds of the invention can be tested for one or more the characteristic IPG-P and/or IPG-A activities mentioned above to determine whether they will be suitable for use a IPG mimetics or antagonists. Preferred assays measure the effect of the compounds on PDH phosphatase, PKA or lipogenesis. Protocols for these assays are provided in Caro et al ^{[14]}. The compounds can also be tested to determine whether they activate or inhibit other enzymes involved in insulin signalling mechanism, such as glucose-6-phosphatase.

### Examples

### General Methods.

All reactions were carried out under an atmosphere of dry argon using oven-dried glassware and freshly distilled and dried solvents. THF and diethyl ether were distilled from sodium benzophenone ketyl. Dichloromethane and acetonitrile were distilled from calcium hydride. TLC was performed on Silica gel GF₂₅₄ (Merck) with detection by charring with phosphomolibdic acid/EtOH. For flash chromatography, Silica Gel (Merck 230-400 mesh) was used. Columns were eluted with positive air pressure. Chromatographic eluents are given as volume to volume ratios (v/v). Routine NMR spectra were recorded with Bruker Avance DPX300 (¹H, 300 MHz), Bruker Avance DRX400 (¹H, 400 MHz), and Bruker Avance DRX500 (¹H, 500 MHz) spectrometers. Chemical shifts are reported in ppm, and coupling constants are reported in Hz. Spectra were referenced to the residual proton or carbon signals of the solvent. High-resolution mass spectra were recorded on a Kratos MS-80RFA 241-MC apparatus. Optical rotations were determined with a Perkin-Elmer 341 polarimeter. Elemental analyses were performed using a Leco CHNS-932 apparatus. The organic extracts were dried over anhydrous sodium sulfate and concentrated *in vacuo.*

The synthesis of D-*chiro*-inositol containing IPG-like compounds bearing complex oligosaccharide structures was envisaged using the trichloroacetimidate derivative **1** as glycosyl donor.

Glycosylation of **1** with **2** afforded pseudodisaccharides **3** in 40% yield (Scheme 1). Selective reductive opening of the benzylidene acetals in **3** with NaBH₃CN-HCl afforded the partially protected derivative **4** in good yield. Thus, compound **4** was used as starting material for the synthesis of some trisaccharidic IPG-like structures as indicated in Schemes 2, 3 and 4. Condensation of **4** with glycosyl donor **5** in ether afforded compound **6** in excellent yield (Scheme 2). Removal of the tert-butyldimethylsilyl group in **6** gave compound **7** in quantitative yield. After deallylation compound **7** was converted into **8** and this into the final pseudotrisaccharide **9** by catalytic hydrogenation.

Benzylation of the pseudotrisaccharide intermediate **7** yielded **10** (Scheme 3) that was deallylated to give **11.** Phosphorylation of **11** using the phosphoramidite procedure gave **12** that was then hydrogenated to be converted into the final pseudotrisaccharide **13** in good yield.

The synthetic approach giving rise to the corresponding IPG-like structure with a β configuration of the terminal D-galactopyranosyl unit is shown in Scheme **4.** Treatment of glycosyl acceptor **4** with trichloroacetimidate **14** gave rise to the fully protected pseudotrisaccharide **15** in good yield. Zemplen deacetylation of **15** followed by conventional benzylation yielded **16** that was deallylated to give **17.** Phosphorylation of **17** gave **18** which was transformed into **19** after catalytic hydrogenation.

The synthesis of pseudopentasaccharide **25** was carried out following the strategy indicated in Schemes 5(I) and 5(II). Condensation of the trisaccharide trichloroacetimidate **20** with acceptor **4** afforded the pseudopentasaccharide derivative **21** in reasonable yield. Removal of the pivaloyl group in **21** followed by conventional benzylation yielded compound **22** in quantitative yield. Deallylation of **22** (Scheme 5(II)) gave **23** phosphorylation of which yielded **24.** Final catalytic hydrogenation of **24** gave rise to the pseudopentasaccharide **25.**

### 1-O-(2-Azido-2-deoxy-3-O-benzyl-4,6-O-benzylidene-α-D-glucopyranosyl)-6-O-allyl-2,3,4,5-tetra-O-benzyl-D-chiro-inositol (3)

A mixture **1** (520 mg, 0.985 mmol) and 2 (382 mg, 0.657 mmol) was dissolved in anhydrous CH₂Cl₂ (6.6 mL) and treated with a solution (2.50 µL) of trimethylsilyl triflate (80 µL) in CH₂Cl₂ (2 mL). The mixture was stirred at room temperature for 1.5 h and then 100 µL of the above solution of TMSOTf was added. After an additional hour with stirring (174 mg, 0.328 mmol) in CH₂Cl₂ (1.5 mL) was added and stirring was continued for 2h. The mixture was the treated with Et₃N, evaporated to dryness and the residue fractionated on column chromatography (Hexane 8: AcoEt 1) to yield 3 (130.5 mg, 49%). ¹H NMR (CDCl₃, 500 MHz): δ 7.47-7.21 (m, 30H, ArH), 5.79 (ddt, *J*₁ = 5.6 Hz, *J*₂ *=* 10.5 Hz, *J*₃ = 17.1 Hz, 1H, OCH₂C*H*=CH₂), 5.51 (s, 1H, CH benzyliden), 5.17 (dd, *J*₁ *=* 1.5 Hz, *J*₂ = 17.2 Hz, I H, OCH₂CH=C*H*H), 5.13 (dd, *J*₁= 1.5 Hz, *J*₂= 10.4 Hz, 1 H, OCH₂CH=CH*H*), 4.97-4.76 (m, 10H, AB System), 4.70 (d, *J* = 3.8 Hz, 1H, H₁), 4.25-4.17 (m, 2H, H₅, + OC*H*HCH=CH₂), 3.99 (t, *J*₁*=* 9.4 Hz, 1H, H_{3'}), 3.97 (m, 1H, O-CH-*H*-CH= CH₂), 3.95 (m, 1H, H_{6'eq}), 3.97-3.74 (m, 6H, ChiroIns), 3.64 (t, *J*₁ *=* 9.3 Hz, 1H, H_{4'}), 3.56 (t, *J*= 10.3 Hz, 1H, H_{6'ax}), 3.49 (dd, *J*₁ *=* 3.7 Hz, *J*₂= 9.8 Hz, 1H, H_{2'}).

### 1-O-(2-Azido-2-deoxy-3,6-di-O-benzyl-α-D-glucopyranosyl)-6-O-allyl-2,3,4,5-tetra-O-benzyl-D-chiro-inositol (4)

To a solution of **3** (716 mg, 0.757 mmol) in THF (19 mL) 4Å molecular sieves were added and the mixture stirred for 30 min. Then a 1M solution of sodium cyanoborohydride in THF (15 mL, 15.14 mmol) and a 1M solution of HCl in ether was added until the evolution of gas ceased. The mixture was then treated with saturated aqueous solution of NaHCO₃ and the organic layer washed with saturated NaCl, dried over Na₂SO₄ and evaporated. The residue was purified by column chromatography (Hexane 4: AcoEt 1) to give 4 (575 mg, 80%). ¹H NMR (CDCl₃, 500 MHz): δ 7.44-7.23 (m, 30H, Ar-H), 5.82 (ddt, *J*₁ = 5.6 Hz, *J*₂ = 10.4 Hz, *J*₃ = 17.2 Hz, 1H, OCH₂C*H*=CH₂), 5.21 (broad dd, *J*₁ = 1.6 Hz, *J*₂ = 17.2 Hz, 1H, OCH₂CH=CH*H*), 5.16 (broad dd, *J*₁*=* 1.6 Hz, *J*₂= 10.4 Hz, 1H, OCH₂CH=C*H*H), 4.96-4.65 (m, 10H, AB System), 4.74 (d, J= 3.6 Hz, 1H, H_{1'}), 4.44 (d, *J=* 12.0 Hz, 1H, AB System), 4.32 *(d, J=* 12.1 Hz, 1H, AB System), 4.22 (broad, dd, *J*₁ = 5.4 Hz, *J*₂ = 13.0 Hz, 1H, OC*H*HCH=CH₂), 4.12 (m, 1H, H₅), 4.0 (m, 1H, OCH*H*CH=CH₂), 4.04-3.78 (m, 6H, ChiroIns), 3.76 (m, 2H, H_{3'} ++H_{4'}), 3.45 (dd, *J*₁*=* 3.6 Hz, *J*₂ *=* 10.0 Hz, 1H, H_{2'}), 3.38 *(dd, J*₁ *=* 3.5 Hz, *J*₂= 10.4 Hz, 1H, H_{6'b}), 3.27(dd, *J*₁ = 4.2 H₄ *J*₂= 10.4 Hz, 1H, H_{6'a}), 2.39 (d, J= 1.6Hz, 1H, OH_{4'}).

### O-(2,3,4-Tri-O-benzyl-6-O-tert-butyl diphenylsilyl-α-D-galactopyranosyl)-(14)-O-(2-azido-3,6-di-O-benZ)'1-2-deoxy-α-D-glucopyranosyl)-(1-6)-1-O-allyl-2,3,4,5-tetra-O-benzyl-D-chiro--inositol (6)

To a solution of **4** (144 mg, 0.152 mmol) and 5 (253 mg, 0.304 mmol) in ether (3 mL) 4Å molecular sieves was added and the mixture stirred at room temperature for 15 min. Then a solution (98 µL) of TMSOTf in ether (40µL in 2 mL) was added and the mixture stirred at room temperature. After 1h **5** (85 mg) in ether (1 mL) was added. After 1h, Et₃N was added and the mixture was filtered, evaporated to dryness and the residue was purified by column chromatography (hexane 8: AcoEt 1) to give 6 (201, 3 mg, 82%). ¹H NMR (CDCl₃, 500 MHz): δ 7.60-7.08 (m, 55H, ArH), 5.79 (m, 1H, OCH₂C*H*= CH₂), 5.50 (d, J= 3.6 Hz, H_{1c}), 5.17 (m, 1H, OCH₂CH= C*H*H), 5.12 (m, 1 H, OCH₂CH= CH*H*), 4.97-4.48 (m, 16H, AB System), 4.75 (d, *J*= 3.7 Hz, H_{1b}), 4.32 (d, *J=* 12.1 Hz, 1H, AB System), 4.23-4.16 (m, 3H, AB System + H_{5b} + OC*H*H=CH₂), 4.05 (t, J= 3.7 Hz, 1H), 4.01-3.78 (m, 12H, OCHHCH= CH₂ + H_{4b} + H_{3b} + H_{2c} + H_{3c} + 7H), 3.73-3.64 (m, 2H), 3.52 (dd, *J*₁= 4.1 Hz, *J*₂= 10.7 Hz, 1H, H_{6b}), 3.45-3.41 (m, 2H, H_{6b} + H_{2b}), 1.06 (s, 9H, ^{t}BuSi).

### O-(2,3,4-Tri-O-benzyl-α-D-galactopyranosyl)-(1-4)-O-(2-azido-3,6-di-O-benzyl-2-deoxy-α-D-glucopyranosyl)-(1-6)-1-O-allyl-2,3,4,5-tetra-O-benzyl-D-chiro-inositol (7)

To a solution of **6** (197.7 mg, 0.122 mmol), 1M solution of TBAF (0.190 mL) was added and the mixture was stirred at room temperature. After 3h, the mixture was treated with ice and extracted with CH₂Cl₂. The organic layer was dried and evaporated and the residue was purified by column chromatography (Hexane 3: AcOEt 1 → Hex 2: AcOEt 1) to give 7 (285 mg, 96%). ¹H NMR (CDCl₃, 500 MHz): δ 7.38-7.11 (m, 45H, ArH), 5.78 (m, 1H, OCH₂C*H*=CH₂), 5.52 (d, *J=* 3.6 Hz, 1H, H_{1c}), 5.18 (m, 1H, OCH₂CH=C*H*H), 5.14 (m, 1H, OCH₂CH=CH*H*), 4.98-4.38 (m, 18H, AB System), 4.76 (d, *J*₁= 4.20 Hz, 1H, H_{1b}), 4.19 (m, 1H, OC*H*HCH=CH₂), 4.14 (m, 1H, H_{5b}), 4.04-3.76 (m, 12H, 6 ChiroIns + H_{4b} + H_{2c} + H_{3b} + H_{3c} + H4_{c} + OCH*H*CH=CH₂), 3.62 (m, 2H, H_{5c} + H_{6b}), 3.52 (m, I H, H_{6c}), 3.48 (dd, *J*₁ = 3.6 Hz, J₂= 10.1 Hz, 1H, H_{2b}, 3.35 (m, 1H, H_{6c}), 3.29 (dd, *J*₁= 1.9 Hz, J₂= 11.4 Hz, 1H, H_{6b}). ¹³C NMR (CDCl₃, 125 MHz): δ 139.03, 139.00, 138.74, 138.69, 138.45, 138.23, 138.04, 134.85, 128.48, 128.46,128.43,128.38,128.35,128.33,128.31,128.29,129.26,127.96,127.86, 127.77, 127.70, 127.63, 127.57, 127.55, 127.47, 127.44, 127.41, 127.27,117.20, 97.99 (anomeric C), 97.16 (anomeric C), 81.94, 81.85, 80.49, 79.94, 78.91, 78.23, 76.13, 75.90, 75.76, 74.94, 74.65, 74.33, 74.22, 74.18, 73.81, 73.75, 73.59, 73.26, 73.00, 72.53, 71.52, 71.04, 68.39, 64.05, 62.20.

### O-(2,3,4-Tri-O-benzyl-α-D-galactopyranosyl)-(1-4)-(2-azido-3,6-di-O-benzyl-2-deoxy-α-D-glucopyranosyl)-(1-6)-2,3,4,5-tetra-O-benzyl-D-chiro-inositol (8)

A solution of the iridium catalyst in anhydrous THF (5.9-10⁻³ M solution, 166 µL) previously treated under a hydrogen atmosphere for 30 minutes was added over a solution of 7 (45 mg, 0.033 mmol) in anhydrous THF (0.33 ml). The mixture was stirred at room temperature for 1.5 h and then THF (1.9 mL), NBS (8.4 mg, 0.047 mmol) and water (116 µL) were added and the mixture was stirred for 5 min, treated with saturated aqueous NaHCO₃, and extracted with CH₂Cl₂. The organic layer was dried and evaporated and the residue was purified by column chromatography (Hexane 2: AcOEt 1) to give pure **8** (41.2 mg, 44%). ¹H NMR (CDCl₃, 500 MHz): δ 7.36-7.11 (m, 45H, ArH), 5.54 (d, *J*= 3.6 Hz, 1H, H_{1c}), 5.01-4.38 (m, 18H, AB System), 4.85 (d, J= 3.8 Hz, 1H, H_{1b}), 4.16-4.14 (m, 2H, H_{5c} + H_{5b}), 4.08-3.78 (m, 10H, ChiroIns x 3 + H_{4b} + H_{2c} + H_{3b} + H_{4c} + H_{3c} + 2H_{6c}), 3.63 (broad t, J= 6.3 Hz, 1H, H₂ₐ), 3.56-3.47 (m, 3H, ChiroIns x 1 + H_{6b} + H_{2b}), 3.35 (m, 1H, H₁ₐ), 3.21 (m, 1H, H_{6b}), 2.71 (s, 1H, OH₁ₐ), 2.53 (broad s, 1H, OH_{6c}), ¹³C NMR (CDCl₃, 125 MHz): δ 138.91, 138.84, 138.65, 138.45, 138.24, 138.11, 138.05, 128.52, 128.46, 128.43, 128.41, 128.37, 128.36, 128.33, 128.31, 128.29, 128.28, 128.26, 128.23, 128.15, 128.00, 127.97, 127.94, 127.92, 127.86, 127.79, 127.69, 127.66, 127.62, 127.54, 127.51, 127.44, 127.40, 127.35, 127.32, 127.27, 127.25, 127.24, 127.22, 97.93 (anomeric C), 97.33 (anomeric C), 81.76, 81.29, 80.45, 80.02, 78.95, 78.33, 76.14, 75.89, 75.72, 75.56, 74.65, 74.33, 74.04, 73.92, 73.75, 73.60, 73.25, 72.98,72.91, 72.52, 72.10, 68.15, 67.32, 63.99, 62.19, 29.73, 29.56.

### O-(α-D-galactopyranosyl)-(1-4)-(2-amino-2-deoxy-α-D-glucopyranosyl)-(1-6)-D-chiro-inositol (9, RGL 1021)

To a solution of **8** (28 mg, 0.021 mmol) in MeOH (4.7 mL), five drops of AcOH and 10% Pd/C (100 mg) were added. The mixture was stirred at room temperature under a hydrogen atmosphere for 2.5 h and then filtered over Celite, washed with methanol and evaporated to give pure **9 (RGL 1021)** (9.3 mg, 88%). ¹H NMR (D₂, 500 MHz): δ 5.50 (broad s, 1H, H_{1c}), 5.36 (broad s, 1H, H_{1b}), 4.33-3.60 (m, 17H), 3.48 (broad d, *J=* 9.0 Hz, H_{2b}).

### O-(2,3,4,6-Tetra-O-benzyl-α-D-galactopyranosyl)-(1-4)-(2-azido-3,6-di-O-benzyl-2-deoxy-α-D-glucopyranosyl)-(1-6)-1-O-allyl-2,3,4,5-tetra-O-benzyl-D-chiro-inositol (10)

Compound **7** (90.6 mg, 0.066 mmol) in DMF (1.3 mL) was treated with sodium hydride (5.25 mg, 0.131 mmol) and benzyl bromide (11.71 µL, 0.098 mmol) at room temperature for 1.5 h. The reaction mixture was cooled to 0 °C, methanol was added and the mixture was extracted with CH₂Cl₂. The extract was washed with saturated aqueous ammonium chloride and then saturated aqueous sodium chloride, dried over Na₂SO₄, evaporated and purified by column chromatography (Hexane 5: AcOEt 1→ Hex 2: AcOEt 1) to give pure **10** (87.5 mg, 91%) as a syrup. ¹H NMR (CDCl₃, 500 MHz): δ 7.40-7.10 (m, 50 H, ArH), 5.79 (m, 1H, OCH₂C*H*= CH₂), 5.52 (d, J= 3.7 Hz, 1H, H_{1c}), 5.17 (m, 1H, OCH₂CH=C*H*H), 5.13 (m, 1H, OCH₂CH=CHH), 5.00-4.62 (m, 14H, AB System), 4.77 (d, J= 3.5 Hz, 1H, H_{1b}), 4.57-4.46 (m, 3H, AB System), 4.33 (d, J= 12.3 Hz, 1H, AB System), 4.24 (d, J= 11.9 Hz, 1H, AB System), 4.19 (d, J= 11.9 Hz, 1H, AB System), 4.17 (m, 2H, H_{5b} + OCHHCH= CH₂), 4.06 (t, *J*= 9.5 Hz, 1H, H_{4b}), 4.01 (m, 1H, H_{2c}), 3.95-3.87 (m, 5H, H_{3b} + H_{4c} + H_{3c} + H_{5c} + OCH*H*CH=CH₂), 4.2-3.75 (m, 6H, ChiroIns), 3.66 (dd, J= 3.1 Hz, J₂= 11.0 Hz, 1H, H_{6b}), 3.47 (m, 2H, H_{2b} + H_{6c}), 3.38 (dd, *J,=* 5.5 Hz, J₂= 8.5 Hz, 1H, H_{6c}), 3.34 (dd, *J*₁= 1.6 Hz, J₂= 10.8 Hz, 1H, H_{6b}). ¹³C NMR (CDCl₃, 125 MHz): δ 138.78, 138.70, 138.61, 138.36, 138.32, 138.15, 137.97, 134.86, 128.36, 128.33, 128.32, 128.29, 128.27, 128.24, 128.22, 128.21, 128.19, 128.02, 127.95, 127.93, 127.91, 127.73, 127.71, 127.61, 127.57, 127.53, 127.48, 127.47, 127.45, 127.39, 127.33, 117.17, 98.40 (anomeric C), 96.98 (anomeric C), 81.94, 81.85, 80.48, 79.92, 78.94, 78.06, 77.31, 77.06, 76.80, 76.08, 75.83, 75.79, 74.83, 74.71, 74.55, 74.47, 74.24, 73.76, 73.70, 73.42, 73.30, 73.02, 72.87, 72.69, 72.49, 70.81, 69.88, 68.55, 64.05.

### O-(2,3,4,6- Tetra-O-benzyl-α-D-galactopyranosyl)-(1-4)-(2-azido-3,6-di-O-benzyl-2-deoxy-α-D-glucopyranosyl-(1-6)-2,3,4,5 tetra-O-benzyl-D-chiro-inositol (11)

A solution of the iridium catalyst in anhydrous THF (5.9 x 10⁻³ M solution, 300 µL) previously treated under a hydrogen atmosphere for 30 minutes was added over a solution of **10** (87 mg, 0.059 mmol) in anhydrous THF (0.6 mL). The mixture was stirred at room temperature for 1h and then THF (3.4 ml), NBS (15.3 mg, 0.086 mmol) and water (208 µL) were added and the mixture was stirred again at room temperature for 10 min, treated with a saturated solution of NaHCO₃. The reaction mixture was then extracted with CH₂Cl₂, washed with saturated NaCl dried over Na₂SO₄ and evaporated. The residue was purified by column chromatography (Hexane 3: AcoEt 1) to give pure 11 (70.8 mg, 84%).¹H NMR (CDCl₃, 500 MHz): δ 7.40-7.12 (m, 50H, ArH), 5.55 (d, J= 3.7 Hz, 1H, H_{1c}), 5.02-4.42 (m, 17H, AB System), 4.85 (d, *J=* 3.7 Hz, 1H, H_{1b}), 4.31 *(d, J=* 12.2 Hz, 1H, AB System), 4.25 (d,*J*= 11.7 Hz, 1H, AB System), 4.20 (d, J= 11.7 Hz, 1H, AB System), 4.19-4.15 (m, 2H, ChiroIns x 1 + H_{5b}), 4.08 (m, 1H, H_{4b}), 4.06 (m, 1H, H₁ₐ), 4.03 (dd, *J*₁ = 3.7 Hz, J₂= 10.2 Hz, H_{2c}), 3.98-3.86 (m, 7H, H_{3c} + H_{5c} + H_{3b} + H_{6b} + Chirolns x 3), 3.80 (t, *J*= 9.2 Hz, 1H, H_{4c}), 3.60 (dd, *J*₁=3.2 Hz, J₂= 11.0 Hz, 1H, ChiroIns), 3.50-3.47 (m, 2H, H_{2b} + ChiroIns), 3.39 (dd, *J*₁ *=* 5.5 Hz, J₂= 8.6 Hz, H_{6b}), 3.28 (dd, *J*₁ *=* 1.8 Hz, J₂= 11.0 Hz, 1H, Chirolns). ¹³C NMR (CDCl₃, 125 MHz): δ 138.95, 138.92, 138.75, 138.67, 138.62, 138.40, 138.35, 138.17, 138.16, 137.98, 137.52, 137.42, 137.40, 137.38, 137.36, 137.35, 137.34, 137.32, 137.31, 137.27, 137.25,137.24, 137.22, 137.21, 137.17, 137.13, 137.11, 137.09, 137.01, 127.99, 127.95, 127.92, 127.73, 127.72, 127.63, 127.55, 127.53, 127.49, 127.48, 127.44, 127.40, 127.38, 127.37, 127.33, 98.30 (anomeric C), 97.25 (anomeric C), 81.76, 81.30, 80.44, 80.04, 78.97, 78.22, 76.09, 75.86, 75.75, 75.30, 74.84, 74.72, 74.20, 74.09, 73.70, 73.43, 73.30, 73.02, 72.78, 72.68, 70.89, 69.84, 68.48, 68.42, 67.30, 64.03.

### O-(2,3,4,6-Tetra-0-benzyl-α-D-galactopyranosyl)-(1-4)-(2-azido-3,6-di-0-benzyl-2-deoxy-α-D-glucopyranosyl)-(1-6)-2,3,4,5-tetra-benzyl-1-O-(dibenzyloxyphosphoryl)-D-chiro-inositol (12)

To a solution of **11** (59 mg, 0.041 mmol) in a 1:1 mixture of dichloromethane-acetonitrile (1 mL), N, *N*-diisopropyl-dibenzyl phosphoramidite (30.5 µL, 0.091 mmol) and tetrazole (13.1 mg, 0.186 mmol) were added and the mixture was stirred for 1h at room temperature. The reaction mixture was cooled to 0 °C and *t*-butyl hydroperoxide (4.7 M isooctane solution, 90 µL) was added and stirring continued for 1 h. The solution was then evaporated to dryness and the residue was purified by column chromatography (Hex 6: AcoEt 1) to give pure 12 as a syrup (67.6 mg, 97%). ¹H NMR (CDCl₃, 500 MHz): δ 7.4-7.1 (m, 55H, ArH), 5.54 (d, J= 3.7 Hz, 1H, H_{1c}), 5.02-4.44 (m, 21H, AB System), 4.87 (dd, *J*₁ = 4.8 Hz, J₂ = 8.4 Hz, 1H, H₁ₐ), 4.79 (d, J= 4.8 Hz, 1H, H_{1b}), 4.33 (d, *J* = 12.2 Hz, 1H, AB System), 4.24 (d, *J=* 11.7 Hz, 1H, AB System), 4.18 (d, *J*= 8.7 Hz, 1H, AB System), 4.13 (t, *J=* 3.8 Hz, 1H, H_{6b}), 4.11-4.04 (m, 3H, H_{5b} + H_{4b} + H₂ₐ), 4.02 (dd, *J*₁ = 3.3 Hz, J₂= 10.3 Hz, 1H, H_{2c}), 3.97 (m, 1H, H_{4c}), 3.91 (broad t, *J=* 6.8 Hz, 1H, H_{5c}), 3.87 (m, 2H, H_{3c} + H_{3b}), 3.83 (t, *J*₁ = 9.1 Hz, 1H, H₄ₐ), 3.74 (t, *J*= 9.5 Hz, 1H, H₃ₐ), 3.71 (dd, *J*₁ *=* 3.3 Hz, J₂= 9.7 Hz, 1H, H₅ₐ), 3.62 (dd, *J*₁ = 2.4 Hz, J₂=11.2 Hz, 1H, H_{6b}), 3.49 (m, 2H, H_{2b} + H_{6c}), 3.38 (dd, *J*₁= 5.3 Hz, J₂= 8.6 Hz, 1H, H_{6c}), 3.28 (broad d, *J=* 9.5 Hz, 1H, H_{6b}). ¹³C NMR (CDCl₃, 125 MHz): δ 138.88, 138.74, 138.71, 138.57, 138.43, 138.30, 138.19, 138.15, 137.99, 137.95, 128.63, 128.58, 128.56, 128.53, 128.48, 128.44, 128.43, 128.40, 128.38, 128.36, 128.34, 128.30, 128.28, 128.25, 128.23, 128.22, 128.19, 128.18, 128.16, 128.12, 128.11, 128.09, 128.05, 128.02, 128.01, 128.00, 127.98, 127.93, 127.87, 127.80, 127.78, 127.73, 127.66, 127.63, 127.56, 127.52, 127.48, 127.46, 127.43, 127.41, 127.37, 127.34, 98.32 (anomeric C), 97.71 (anomeric C), 81.37, 81.02, 80.68, 79.00, 77.92, 77.88, 77.50, 77.26, 76.12, 75.90, 75.75, 74.85, 74.73, 74.71, 74.66, 74.30, 74.02, 73.89, 73.44, 73.08, 72.73, 72.70, 72.65, 72.62, 72.40, 71.17, 69.81, 69.59, 69.55, 69.33, 69.28, 69.26, 69.21, 68.46, 68.42, 64.10.

### O-α-D-galactopyranosyl-(1-4)-2-ammonio-2-deoxy-α-D-glucopyranosyl-(1-6)-D-chiro-inositol-1-phosphate (13, RGL 1022)

To a solution of **12** (62.8 mg, 0.037 mmol) in methanol (4.3 mL) 10% Pd on C (176 mg), AcOH/AcONa buffer (0.2 M, pH 5, 4.3 mL) and THF (0.6 mL) were added. The mixture was stirred under a hydrogen atmosphere for 24 h and then filtered a lyophilised to give **13**. ¹H NMR (D₂O, 500 MHz): δ 5.49 (broad s, 1H, H_{1c}), 5.12 (broad s, 1H, H_{1b}), 4.53 (m, 1H, H₁ₐ), 4.26-3.6 (m, 17H). ³¹P NMR (D₂O, 202 MHz): δ 3.38.

### O-(2,3,4,6-Tetra-O-acetyl-β-D-galactopyranosyl)-(1-4)-(2-azido-3,6-di-O-benzyl-2-deoxy-α-D-glucopyranosyl)-(1-6)-1-O-allyl-2,3,4,5-tetra-O-benzyl-D-chiro-inositol (15)

To a solution of compound **14** (36.3 mg, 0.074 mmol) and compound **4** (38.8 mg, 0.041 mmol) in anhydrous ether (0.5 mL) powdered 4Å molecular sieves were added and the mixture was stirred for 30 min at room temperature. Then TMSOTf (0.11 M solution in ether, 33 µL) was added and the mixture was stirred at room temperature for 45 min. The mixture was treated with Et₃N, filtered and evaporated. The residue was fractionated by column chromarography (Hexane 3: AcoEt 1) to give **15** (36.8 mg, 70%). ¹H NMR (CDCl₃, 500 MHz): 7.43-7.21 (m, 30H, ArH), 5.81 (m, 1H, OCH₂C*H*=CH₂), 5.20 (m, 1H, OCH₂CH=C*H*H), 5.19 (m, 1H), 5.14 (m, 1H, OCH₂CH=CH*H*), 5.05 (d, *J*= 10.5 Hz, 1H, AB System), 5.02 (dd, *J*₁= 10.5 Hz, J₂= 8.1 Hz, 1H, H_{2c}), 4.96-4.89 (m, 3H, AB System), 4.79 (m, 2H, AB System), 4.73 (d, J= 3.8 Hz, 1 H, H_{1b}), 4.69-4.62 (m, 6H, H_{5c} + H_{3c} + H_{6c} + 3H), 4.25 (d, J= 8.2 Hz, 1H, H_{1c}), 4.21 (m, 1H, OC*H*HCH= CH₂), 4.18 (d, J= 12.2 Hz, 1H, AB System), 4.0-3.71 (m, 12H, OCH*H*CH= CH₂+ H_{6b}+ H_{4b} + H₃ₐ + H_{5b} + H_{3b} + 6H), 3.47 (dd, *J*₁= 3.7 Hz, J₂= 10.1 Hz, 1H, H_{2b}), 3.42 (m, 2H), 3.17 (broad d, J= 10.8 Hz, 1H, Hₐ), 2.07 (s, 3H, CH₃COO), 1.98 (s, 3H, CH₃COO), 1.92 (s, 3H, C*H*₃COO), 1.69 (s, 3H, CH₃COO). ¹³C NMR (CDCl₃, 125 MHz): δ 170.19, 170.15, 170.02, 168.94, 139.06, 138.89, 138.75, 138.70, 138.26, 137.40, 134.87, 128.71, 128.40, 128.36, 128.32, 128.20, 128.14, 127.69, 127.63, 127.56, 127.54, 127.45, 127.24, 99.85 (anomeric C), 97.75 (anomeric C), 81.94, 81.71, 80.02, 78.88, 78.17, 77.28, 77.03, 76.77, 76.11, 76.07, 75.84, 75.70, 75.20, 73.95, 73.54, 73.42, 73.26, 72.61, 70.90, 70.54, 70.40, 69.41, 66.88, 66.74, 63.16, 60.94, 60.45, 20.67, 20.62, 20.56, 20.51,.

### O-(2,3,4,6- Tetra-O-benzyl-β-D-galactopyranosyl)-(1-4)-(2-azido-3,6-di-O-benzyl-2-deoxy-α-D-glucopyranosyl(1-6)-1-O-allyl-2,3,4,5-tetra-O-benzyl-D-chiro-inositol (16)

To a solution of **15** (32 mg, 0.025 mmol) in MeOH (0.5 mL) a 1M solution of sodium methoxide (20 µL) was added and the mixture stirred for 20 min at room temperature.

Then the mixture was evaporated to dryness, toluene was added and evaporated. The residue was solved in DMF (0.5 mL) and sodium hydride (8 mg, 0.2 mmol) and benzyl bromide (18 µL, 0.15 mmol) were added. The mixture was stirred overnight at room temperature and then cooled to 0 °C, methanol was added and the mixture was extracted with AcOEt. The extract was washed with saturated aqueous ammonium chloride, and saturated aqueous sodium chloride, dried over Na₂SO₄ and evaporated. The residue was purified by column chromatography (Hexanes: AcOEt 1) to give pure **16** (31.5 mg, 86%). ¹H NMR (CDCl₃, 500 MHz): δ 7.38-7.06 (m, 50H, ArH), 5.74 (m, 1H, OCH₂CH= CH₂), 5.15 (m, 1H, OCH₂CH=CHH), 5.12 (d, J= 10.5 Hz, 1H, AB System), 5.10 (m, 1H, OCH₂CH= CH*H*), 4.96-4.59 (m, 14H, AB System), 4.71 (d, J= 4.1 Hz, 1H, H_{1b}), 4.49 (m, 2H, AB System), 4.31 (d, J= 12.3 Hz, 1H, AB System), 4.25 (d, J= 7.7 Hz, 1H, H_{1c}), 4.20 (m, 2H, AB System), 4.17-4.12 (m, 2H, OCHHCH=CH₂ + 1H), 4.04 (t, J= 9.0 Hz, 1H, Hₐ), 3.97 (dd, J₁= 3.1 Hz, J₂= 9.7 Hz, 1H, Hₐ), 3.93 (m, 1H, H_{4b}) 3.9 (m, 1H, OCHHCH= CH₂), 3.86-3.71 (m, 7H, H_{3b} + H_{2c} + 4Hₐ + 1H), 3.45 (t, J= 8.6 Hz, 1H, H_{4c}), 3.42 (dd, *J*₁= 3.6 Hz, J₂= 10.3 Hz, 1H, H_{2b}), 3.3-3.21 (m, 4H, H_{3c} + H_{5c} + 2H).

### O-(2,3,4,6-Tetra-O-benzyl-β-D-galactopyranosyl)-(1-4)-(2-azido-3,6-di-O-benzyl-2-deoxy-α-D-glucopyranosyl)-(1-6)-2,3,4,5-tetra-O-benzyl-D-chiro-inositol (17)

A solution of the iridium catalyst in anhydrous THF (5.9 x 10⁻³ M solution, 92 µL) previously treated under a hydrogen atmosphere for 30 min was added to a solution of **16** (26.1 mg, 0.018 mmol) in anhydrous THF (0.18 mL). The mixture was stirred to room temperature for 1.5 h and cooled to 0 °C THF (1 mL), NBS (4.58 mg, 0.025 mmol) and water (60 µL) were added and the mixture was stirred at 0 °C for 20 min. Then saturated aqueous NaHCO₃ was added and the mixture extracted with CH₂Cl₂. The extract was washed with saturated aqueous NaCl, dried and evaporated. The residue was purified by column chromatography (Hexane 3: AcOEt 1**→** Hex 2: AcOEt 1) to give pure 17 (21.1 mg, 83%). ¹H NMR (CDCl₃, 500 MHz): δ 7.36-7.07 (m, 50H, ArH), 5.13 (d, J= 10.1 Hz, 1H, AB System), 4.89-4.60 (m, 13H, AB System), 4.78 *(d, J=* 3.8 Hz, 1H H_{1b}), 4.48 (m, 2H, AB System), 4.32 *(d, J=* 11.7 Hz, 1H, AB System), 4.24 (d, *J=* 7.7 Hz, 1H,H_{1c}), 4.18 (m, 2H, AB System), 4.12-4.04 (m, 5H, AB System x 2 + H_{5c} + H_{4b} + Hₐ), 3.99 (dd, *J*₁= 2.2, J₂= 9.5 Hz, 1H, Hₐ), 3.87-3.68 (m, 8H, H_{4c}+ H_{3b}+ H_{2c}+ H_{5b} + H₁ₐ + H_{6c} + 2H), 3.45 (t, *J=* 8.6 Hz, 1H, Hₐ), 3.42 (dd. *J*₁*=* 3.8 Hz, J₂= 10.1 Hz, 1H, H_{2b}), 3.3-3.23 (m, 3H, H_{3c} + 2Hₐ), 3.12 (broad d, J= 10.2 Hz, 1H, H_{6c}), 1.83 (s, 1H, OH₁ₐ). ¹³C NMR (CDCl₃, 125 MHz): δ 139.07, 138.74, 135.71, 138.66, 138.57, 138.49, 138.44, 138.26, 138.14, 137.99, 128.63, 128.51, 128.42. 128.37, 128.35, 128.33, 128.29, 129.19, 128.16, 127.98, 127.93, 127.83, 127.80, 127.77, 127.76, 127.74, 127.73, 127.71, 127.67, 127.64, 127.61, 127.58, 127.52, 127.50, 127.48, 127.44, 127.39, 127.37, 127.34, 127.27, 102.66 (anomeric C), 98.40(anomeric C), 82.36, 81.69, 81.36, 80.06, 79.67, 78.26, 76.78, 76.61, 76.20, 76.01, 75.71, 75.25, 75.03, 74.75, 73.76, 73.40, 73.21, 73.13, 73.02, 72.72, 72.68, 71.09, 68.12, 67.99, 67.52, 67.36, 63.28, 25.64.

### O-(2,3,4,6-Tetra-O-benzyl-(3-D-galactopyranosyl)-(1-4)-(2-azido-3,6-di-O-benzyl-2-deoxy-a-D-glucopyranosyl)-(1-6)-2,3,4,5-tetra-O-benzyl-1-O-(dibenzyloxyphosphoryl)-D-chiro-inositol (18)

To a solution **17** (21 mg, 0.015 mmol) in a 1:1 mixture of CH₂Cl₂: CH₃CN (0.4 mL), N, N-diisopropyl dibenzyl phosphoramidite (16 µL, 0.046 mmol) and tetrazole (8 mg, 0.112 mmol) was added and the mixture was stirred for 3h at room temperature. The reaction mixture was then cooled to 0°C and t-butyl hydroperoxide (4.7 M isooctane solution, 34 µL) was added and stirring was continued for 1h. The solution was then evaporated to dryness and the residue was purified by column chromatography (cyclohexane 3: AcOEt 1) to give **18** (39%). ¹H NMR (CDCl₃, 500 MHz): δ 7.39-7.04 (m, 60H, ArH), 5.09 (d, J= 10.1 Hz, 1H, AB System), 4.97-4.44 (m, 20H, AB System), 4.88 (d, J= 4.0 Hz, 1H, H₁ₐ), 4.73 (d, *J=* 3.8 Hz, H_{1b}), 4.31 (d, *J=* 12.0 Hz, 1H, AB System), 4.27 (d, J= 7.7 Hz, 1H, H_{1c}), 4.19 (m, 2H, AB System), 4.13-4.01 (m, 4H, H₂ₐ + 1Hₐ + 2H_{b}), 3.85 (m, 1H, 1H_{c}), 3.80-3.67 (m, 6H, H_{3b}+ H_{2c} + 3Hₐ + 1H), 3.47-3.39 (m, 2H, H_{2b} + 1H), 3.30-3.39 (m, 2H, H_{2b} + 1H), 3.30-3.23 (m, 3H, 1H_{c} + 2H), 3.14 (m, 1H, 1H_{b}). ³¹P NMR (CDCl₃, 202 MHz): δ -2.20.

### O-β-D-gatactopyranosyl-(1-4)-2-ammonio-2-deoxy-α-D-galactopyranosyl-(1-6)-D-chiro-inositol-1-phosphate (19)

To a solution of **18** (3.3 mg, 1.9 µmol) in methanol (0.22 mL) AcOH/AcONa buffer (0.2 M, pH5, 0.22 mL) and 10% Pd/C (5 mg) were added. The mixture was stirred under a hydrogen atmosphere for 2 h and then filtered and liophylized. The residue was passed through a sephadex G-10 column (10% EtOH in water) to give pure **19**. ¹H NMR (D₂O, 500 MHz): δ 5.13 (broad s, 1H, H_{1b}), 4.56 (m, 1H, H₁ₐ), 4.50 (d, *J=* 7.5 Hz, 1H, H_{1c}), 4.24-3.57 (m, 17H). ³¹P NMR (D₂O, 202 MHz): δ 3.36.

### O-(3,4,6-Tri-O-benzyl-2-O-pivaloyl-α-D-mannopyranosyl)-(1-2)-(3,4,6-tri-O-benzyl-α-D-mannopyranosyl)-(1-6)-(2,3,4-tri-O-benzyl-α-D-mannopyranosyl)-(1-4)-(2-azido-3,6-di-O-benzyl-2-deoxy-α-D-glycopyranosyl)-(1-6)-1-O-allyl-2,3,4,5-tetra-O-benzyl-D-chiro-inositol (21)

To a mixture **20** (104 mg, 0.067 mmol), 4 (89.41 mg, 0.094 mmol) and 4Å molecular sieves in CH₂Cl₂ (2 mL) at room temperature was added TMSOTf (1.0 µL, 0.005 mmol). After 1.5 h at room temperature the reaction mixture was neutralized with Et₃N, filtered and evaporated to dryness. The residue was purified on column chromatography (cyclohexane 10: AcOEt 1) to give **21** (62.2 mg, 58%). ¹H NMR (CDCl₃, 500 MHz): δ 4.41-7.06 (m, 75 H, ArH), 5.86-5.77 (m, 1H, OCH₂C*H*=CH₂), 5.48 (dd, *J=* 3.1 Hz, J₂= 2.2 Hz, 1H, H_{2c}), 5.26 (d, 1H, *J*= 2.0 Hz, H_{2d}), 5.21-5.12 (m, 2H, OCH₂CH=C*H*₂), 5.01 (d, 1H, J= 2.0 Hz, H_{1c}), 4.96-3.34 (m, 63H), 1.72 (s, 9H, ^{t}Bu).

### O-(2,3,4,6-Tetra-O-benzyl-α-D-mannopyranosyl)-(1-2)-(3,4,6-tri-O-benzyl-α-D-mannopyranosyl)-(1-6)-(2,3,4,6-tri-O-benzyl-α-D-mannopyranosyl)-(1-4)-(2-azido-3,6-di-O-benzyl-2-deoxy-α-D-glucopyranosyl)-(1,-6)-1-O-allyl-2,3,4,5-tetra-O-benzyl-D-chiro-inositol (22)

To a solution of **21** (97.3 mg, 0.042 mmol) in 1:1 methanol: THF (1.35 mL), sodium methoxide in methanol (1M solution, 94 µL) was added and the mixture was stirred overnight at room temperature. The solution was evaporated to dryness, toluene was added to the residue and evaporated. The residue was solved in DMF (1.35 mL) and NaH (3.3 mg) and benzyl bromide (7.5 µL) were added. The mixture was stirred at room temperature for 24 h, cooled to 0 °C, treated with MeOH and extracted with CH₂Cl₂. The extract was washed with sat. NH₄Cl, sat. NaCl, dried over Na₂SO₄ and evaporated. The residue was purified by column chromatography (Hexane 6: AcoEt 1) to give pure **22** (99%). ¹H NMR (CDCl₃, 500 MHz): δ 7.38-7.09 (m, 80H, ArH), 5.82 (m, 1H, OCH₂*CH*=CH₂), 5.28 (d, J= 2.1 Hz, 1H, H_{1d}), 5.23-5.12 (m, 3H, OCH₂CH=C*H*₂ + 1H_{d}), 4.98-4.76 (m, 9H, AB System), 4.90 (m, 1H, 1H_{d}), 4.70 (d, J= 3.6 Hz, 1H, 1H_{b}), 4.70-3.35 (m, 55H).

### O-(2,3,4,6-Tetra-O-benzyl-α-D-mannopyranosyl)-(1-2)-(3,4,6-tri-O-benzyl-α-D-mannopyranosyl)-(1-6)-(2,3,4-tri-O-benzyl-α-D-mannopyranosyl)-(1-4)-(2-azido-3,6-di-O-benzyl-2-deoxy-α-D-glucopyranosyl)-2,3,4,5-tetra-O-benzyl-D-chiro-inositol (23)

A solution of the iridium catalyst in anhydrous THF (5.9 x 10⁻³ M, solution, 88 µL) previously treated under a hydrogen atmosphere for 30 min was added over a solution of **22** (40 mg, 0.071 mmol) in THF (0.2 mL). The mixture was stirred for 45 min and THF (1 mL), NBS (4.38 mg, 0.025 mmol) and water (60 µL) were added and the mixture was stirred for 5 min, treated with saturated NaHCO₃, and extracted with CH₂Cl₂. The extract was dried over Na₂SO₄ and evaporated. The residue was purified by column chromatography (Hexane 4: AcOEt 1→ Hexane 2: AcOEt 1) to give 23 (93%). ¹H NMR (CDCl₃, 500 MHz): δ 7.35-7.06 (m, 80H, ArH), 5.26 (d, J= 2.2 Hz, 1H, 1H_{d}), 5.11 (d, J= 2.0 Hz, 1H, 1H_{d}), 4.95 (m, 1H, AB System), 4.86 (d, J= 2.0 Hz, 1H, 1H_{d}), 4.89-3.2 (m, 62H).

### O-(2,3,4,6-Tetra-O-benzyl-α-D-mannopyranosyl)-(1-2)-(3,4,6-tri-O-benzyl-α-D-mannopyranosyl)-(1-6)-(2,3,4-tri-O-benzyl-α-D-mannopyranosyl)-(1-4)-(2-azido-3,6-di-O-benzyl-2-deoxy-α-D-glucopyranosyl)-2,3,4,5-tetra-O-benzyl-1-O-(dibenzylphosphoryl)-D-chiro-inositol (24)

To a solution of **23** (12.8 mg, 0.005 mmol) in a 1:1.5 mixture of CH₂Cl₂: CH₃Cl (1.6 mL), N, N-diisopropyl dibenzyl phospharamidite (22 µL, 0.067 mmol) and tetrazole (5.7 mg, 0.080 mmol) was added and the mixture stirred for 45 min at room temperature. The mixture was cooled to 0 °C and t-butyl hydroperoxide (4.7M isooctane solution, 50 µl) was added and stirring was continued for 30 min. The mixture was evaporated to dryness and the residue was purified by column chromatography (Hexane 1: Ether 2) to give **24** (60%). ¹H NMR (CDCl₃, 500 MHz): δ 7.40 (m, 90H, ArH), 5.27 (d, *J=* 2.3 Hz, 1H, 1H_{d}), 5.11 (d, J= 2.0 Hz, 1H, 1H_{d}), 4.96-3.26 (m, 68H).

### O-α-D-Mannopyranosyl-(1-2)-O-α-D-mannopyranosyl-(1-6)-O-α-D-mannopyranosyl-(1-4)-O-2 ammonio-2-deoxy-α-D-glucopyranosyl-(1-6)-D-chiro-inositol-1-phosphate (25)

A solution of **24** (3 mg) in THF-EtOH (1:11) (50 µL) containing NH₄OAc (0.5 mg) was stirred for 12h under atmospheric pressure of H₂ with 10% Pd/C, the filtered over Celite and concentrated. The crude mixture was passed through Sephadex G.25 eluting with H₂-EtOH (10:1). Liophylisation gave **25** as a white powder. ¹H NMR (CDCl₃, 500 MHz): δ 5.26 (bs, 1H), 5.17 (bs, 1H), 5.06 (bs, 1H), 4.8 (bs, 1H), 4.52-3.54 (m, 29H), 2.97 (bs, 1H).

| **Assay Data** | |
|---|---|
| **PDH activation at 100µM** | |
| RGL1021 | 14% |

| **PKA inhibition at 0.1µM** | |
|---|---|
| RGL1022 | 47% |

### References:

[1](a) Varela-Nieto et al, Comp. Biochem. Physiol.,. 115B:223-241, 1996; (b) Strälfors, Bioassays, 19:327-335, 1997; (c) Field, Glycobiology, 7:161-168, 1997; d) Jones & Varela-Nieto, Int. J. Biochem. Cell Biol., 30:313-326, 1998.
[2] Mato et al, Biochem. Biophys. Res. Commun., 146:746-770, 1987.
[3] Larner et al, Biochem. Biophys. Res. Commun., 151:1416-1426, 1998.
[4] Caro et al, Biochem. Mol. Med., 61:214-228, 1997.
[5] For recent reviews on the synthesis of these structures see: a) Gigg & Gigg in Glycopeptides and Related Compounds, Large & Warren, Eds., Marcel Dekker, New York, 1997, pp 327-392; Khiar & Martin-Lomas in Carbohydrate Mimics. Concepts and Methods, Chapleur Ed Wiley VCH, 1998, pp 443-462; Dietrich et al, Chem. Eur. J., 5:320-336, 1999.
[6] Jaramillo et al, J. Org. Chem., 59, 3135-3141, 1994.
[7] Corey & Venkateswarlu, J. Am. Chem. Soc., 94:6190, 1974.
[8] Ley et al, Angew.Chem. Int. Ed. Engl., 33:2290-2292, 1994.
[9] Kinzi & Schmidt, Liebigs Ann. Chem., 1537-1545, 1985.
[10] Vasella et al, Helv. Chim. Acta., 74:2073-2077, 1991.
[11] Schmidt & Kinzi, Adv. Carbohydy. Chem. Biochem., 50:21-123, 1994.
[12] Once et al, Chem. Eur. J., 3:431-440, 1997.
[13] Rademacher et al, Brazilian J. Med. Biol. Res., 27:327-341, 1994.
[14] Caro et al, Biochem. Molec. Med., 61:214-228, 1997.
[15] Kunjara et al, In: Biopolymers and Bioproducts: Structure, Function and Applications, Ed Svati et al, 301-305, 1995.
[16] Zapata et al, Carbolaydrate Res., 264:21-31, 1994.
[17] Dietrich et al, Chem. Eur. J.,5:320-336, 1999.
[18] Baeschlin et al, Chem. Eur. J., 6(1):172-186, 2000.
WO98/11116 and WO98/11117 (Rademacher Group Limited).
WO98/11435 and WO98/10791 (Rademacher Group Limited).
WO99/38516 (Rademacher Group Limited).

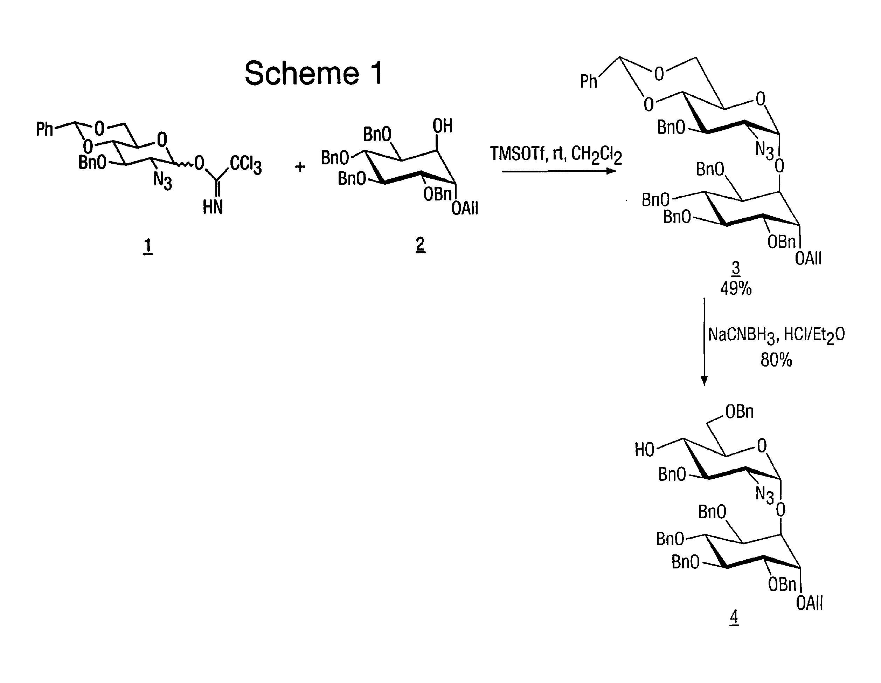

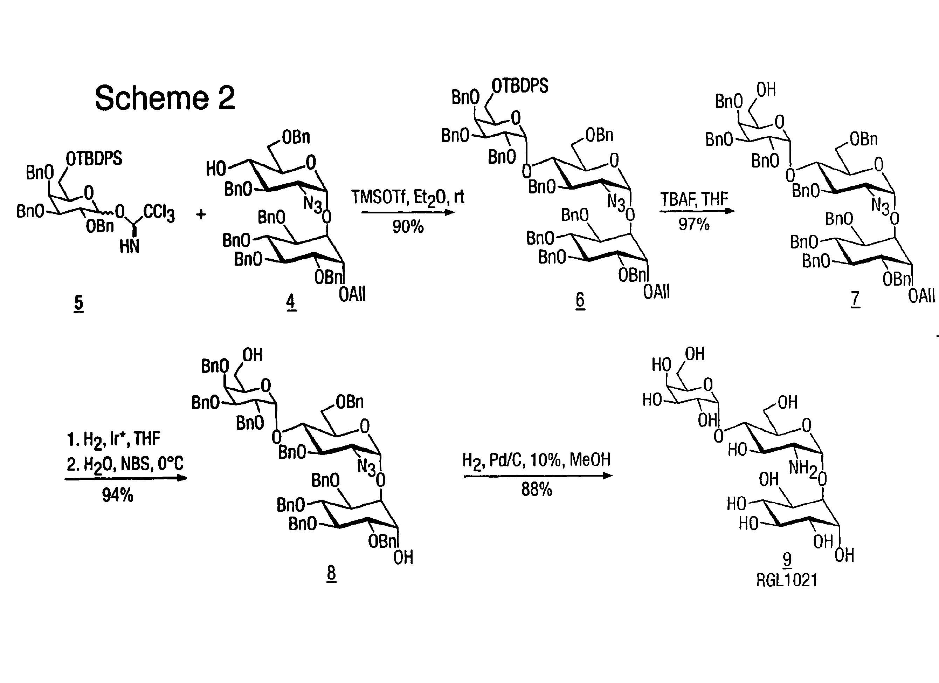

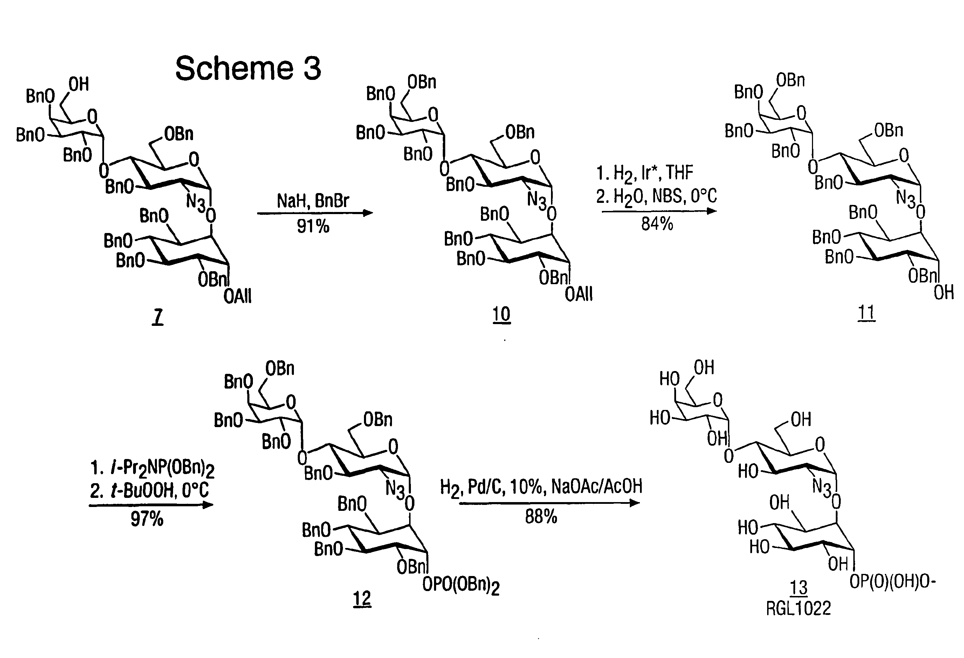

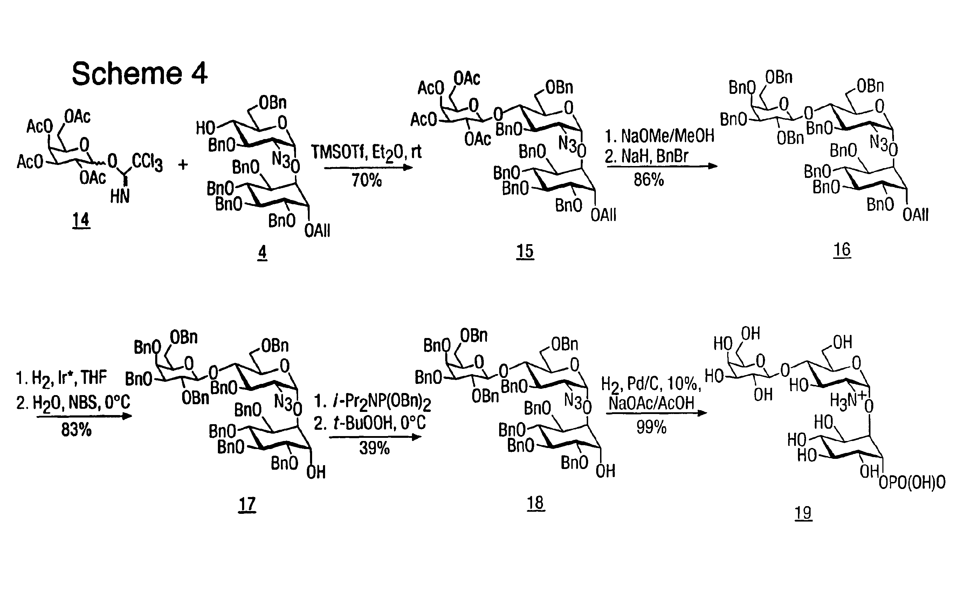

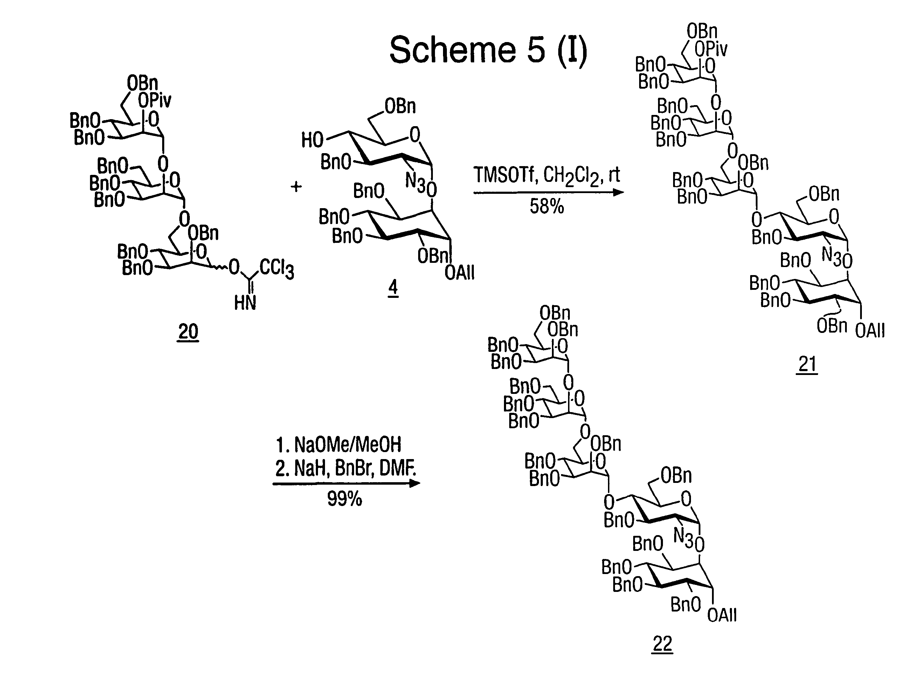

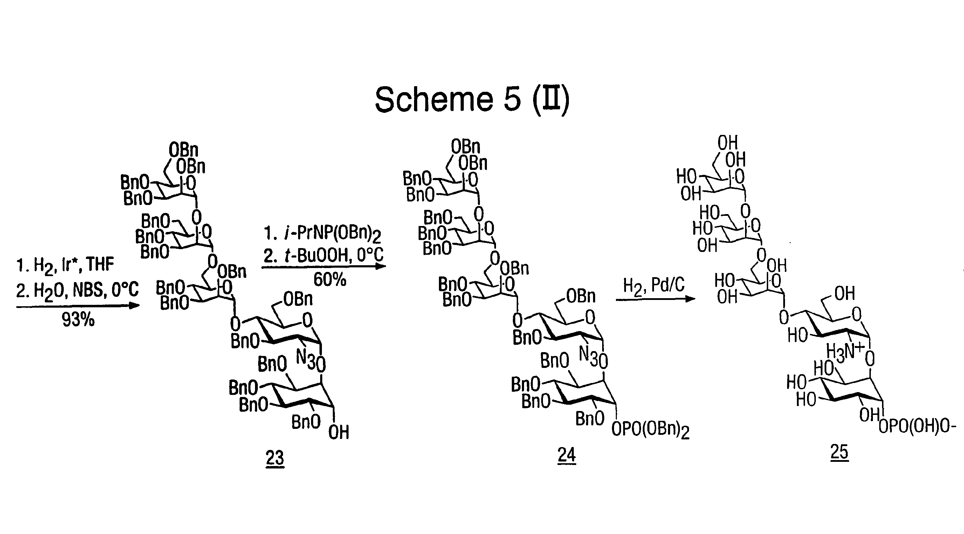

## Claims

1. A compound represented by the general formula:
Y-X-1,6-chiro-inositol
wherein:
X represents a sugar radical;
Y represents one to three sugar radicals;
the sugar residue is unsubstituted or substituted with between one and four groups, and the cyclitol is unsubstituted or is further substituted with between one and four groups, the group or groups being independently selected from:
(a) a phosphoryl group which is phosphate -O-P(O)(OH)₂; thiophosphate -OP(S)(OH)₂; phosphate esters -O-P(O)(OR)₂; thiophosphate esters -OP(S)(OR)₂; phosphonate -O-P(O)OHR; thiophosphonate -O-P(S)OHR; substituted phosphonate -O-P(O)OR₁R₂; substituted thiophosphonate -OP(S)OR₁R₂; -O-P(S)(OH)(SH); or cyclic phosphate;
(b) a phosphorus containing compound which is phosphoramidite -O-P(OR)-NR₁R₂ phosphoramidate -O-P(O)(OR)-NR₁R₂;
(c) a sulphur groups which is -O-S(O)(OH), -SH, -SR, -S(→O)-R, -S(O)₂R, RO-S(O)₂⁻, -O-SO₂NH₂, -O-SO₂R₁R₂ or sulphamide -NHSO₂NH₂;
(d) an amino group which is -NHR, -NR₁R₂, -NHAc, -NHCOR, -NH-O-COR,-NHSO₃⁻, -NHSO₂R or -N(SO₂R)₂, and/or an amidino group which is -NH-C(=NH)NH₂ and/or an ureido group which is -NH-CO-NR₁R₂ or a thiouriedo group which is -NH-C(S)-NH₂;
(e) a hydroxy group or substituted hydroxy group which is -OR₃, where R₃ is C₁₋₁₀ unsubstituted or substituted alkyl, e.g. CHF₂ or CF₃, alkoxyalkyl, aryloxyalkyl, cycloalkyl, alkenyl (unsubstituted alkyl), alkylene (C₃₋₇ cycloalkyl), -OCOR, aryl, heteroaryl, acetal, or where two hydroxyl groups are joined as a ketal;
(f) a halogen substituent which is fluorine or chlorine;
(g) a hydrogen, to provide a deoxy sugar;
wherein R, R₁ and R₂ are independently hydrogen or C₁₋₁₀ unsubstituted or substituted alkyl or aryl;
or a salt coordination complex with metal ions, ester, free acid or base, hydrate thereof, or a glycolipid derivative in which a lipid moiety cleavable by a phospholipase enzyme is a substituent of a sugar residue or the cyclitol.

2. The compound of claim 1, wherein the sugar residue and chiro-inosotol are α linked.

3. The compounds of claim 1, wherein the sugar residue and chiro-inosotol are β linked.

4. The compound of any one of the preceding claims, wherein the sugar residue is a hexose or a pentose, or substituted forms thereof.

5. The compound of claim 4, wherein the sugar residue is a hexose selected from the group consisting of glucose, galactose or mannose.

6. The compound of claim 4, wherein the sugar residue is a hexosamine.

7. The compound of claim 6, wherein the hexosamine is galactosamine or glucosamine.

8. The compound of any one of the preceding claims, wherein the chiro-inositol is a D or L-enantiomer.

9. The compound of any one of the preceding claims which is:
RGLI021 *O*-(D-galactopyranosyl)-α(1,4)-*O*-(2'-amino-2'-deoxy-D-glucanopyranosyl)-α(1,6)-*chiro*-inositol;
RGL1022 *O*-(D-galactopyranosyl)-α(1,4)-*O*-(2'-amino-D-glucanopyranosyl)-α(1,6)-*chiro*-inositol-1-phosphate;
Compound 25 *O*-α-D-Mannopyranosyl-(1,2)-*O*-α-D-mannopyranosyl-(1,6)-*O*-α-D-mannopyranosyl-(1,4)-*O*-2 ammonio-2-deoxy-α-D-glucopyranosy)-(1,6)-D-*chiro*-inositol 1-phosphate; and
Compound 19 O-β-D-galactopyranosyl-(1-4)-2-aminonio-2-deoxy-α-D-galactopyranosyl-(1,6)-D-*chiro*-inositol-1-phosphate;
or a salt, coordination complex with metal ions, ester, free acid or base, hydrate thereof, or a glycolipid derivative in which a lipid moiety cleavable by a phospholipase enzyme is a substituent of a sugar residue or the cyclitol.

10. A composition comprising a compound of any one of the preceding claims, in combination with a pharmaceutically acceptable carrier.

11. A compound of any one of claims 1 to 9 for use in therapy.

12. Use of a compound of any one of claims 1 to 9 for the preparation of a medicament for the treatment of a condition ameliorated by the administration of an inositol phosphoglycan (IPG) second messenger or an IPG antagonist.

## Patentansprüche

1. Verbindung der allgemeinen Formel:
Y-X-1,6-chiro-Inosit
worin:
X für einen Zuckerrest steht;
Y für ein bis drei Zuckerreste steht;
der Zuckerrest unsubstituiert oder mit zwischen ein und vier Gruppen substituiert ist und der Cyclit unsubstituiert oder mit zwischen ein und vier Gruppen weiter substituiert ist, wobei die Gruppen unabhängig voneinander ausgewählt sind aus:
(a) einer Phosphorylgruppe, die Phosphat -O-P(O)(OH)₂; Thiophosphat -O-P(S)(OH)₂; ein Phosphatester -O-P(O)(OR)₂; ein Thiophosphatester -O-P(S)(OR)₂; Phosphonat -O-P(O)OHR; Thiophosphonat -O-P(S)OHR; substituiertes Phosphonat -O-P(O)OR₁R₂; substituiertes Thiophosphonat -O-P(S)OR₁R₂; -O-P(S)(OH)(SH); oder zyklisches Phosphat ist;
(b) einer phosphorhältigen Verbindung, die Phosphoramidit -O-P(OR)-NR₁R₂ oder Phosphoramidat -O-P(O)(OR)-NR₁R₂ ist;
(c) einer Schwefelgruppe, die -O-S(O)(OH), -SH, -SR, -S(→O)-R, -S(O)₂R, RO-S(O)₂ , -O-SO₂NH₂, -O-SO₂R₁R₂ oder Sulfamid -NHSO₂NH₂ ist;
(d) einer Aminogruppe, die -NHR, -NR₁R₂, -NHAc, -NHCOR, -NH-O-COR, -NHSO₃⁻, -NHSO₂R oder -N(SO₂R)₂ ist, und/oder einer Amidinogruppe, die -NH-C(=NH)NH₂ ist, und/oder einer Ureidogruppe, die -NH-CO-NR₁R₂ ist, oder einer Thioureidogruppe, die -NH-C(S)-NH₂ ist;
(e) einer Hydroxygruppe oder einer substituierten Hydroxygruppe, die -OR₃ ist, worin R₃ unsubstituiertes oder substituiertes C₁₋₁₀-Alkyl, z.B. CHF₂ oder CF₃, -Alkoxyalkyl, -Aryloxyalkyl, -Cycloalkyl, -Alkenyl (unsubstituiertes Alkyl), -Alkylen (C₃₋₇-Cycloalkyl), -OCOR, -Aryl, -Heteroaryl oder -Acetal ist oder worin zwei Hydroxylgruppen als Ketal verbunden sind;
(f) einem Halogensubstituenten, der Fluor oder Chlor ist;
(g) Wasserstoff, um einen Desoxyzucker bereitzustellen;
worin R, R₁ und R₂ unabhängig voneinander Wasserstoff oder unsubstituiertes oder substituiertes C₁₋₁₀-Alkyl oder -Aryl sind;
oder ein Salz, ein Koordinationskomplex mit Metallionen, ein Ester, eine freie Säure oder Base, ein Hydrat davon oder ein Glykolipid-Derivat, in dem eine von einem Phospholipase-Enzym spaltbare Lipidgruppierung ein Substituent des Zuckerrests oder des Cyclits ist.

2. Verbindung nach Anspruch 1, worin der Zuckerrest und der chiro-Inosit α-verbunden sind.

3. Verbindung nach Anspruch 1, worin der Zuckerrest und der chiro-Inosit β-verbunden sind.

4. Verbindung nach einem der vorangegangenen Ansprüche, worin der Zuckerrest eine Hexose oder eine Pentose oder eine substituierte Form davon ist.

5. Verbindung nach Anspruch 4, worin der Zuckerrest eine Hexose ist, die aus der aus Glucose, Galactose und Mannose bestehenden Gruppe ausgewählt ist.

6. Verbindung nach Anspruch 4, worin der Zuckerrest ein Hexosamin ist.

7. Verbindung nach Anspruch 6, worin das Hexosamin Galactosamin oder Glucosamin ist.

8. Verbindung nach einem der vorangegangenen Ansprüche, worin der chiro-Inosit ein D- oder L-Enantiomer ist.

9. Verbindung nach einem der vorangegangenen Ansprüche, die Folgendes ist:
RGL1021 O-(D-Galactopyranosyl)-α(1,4)-O-(2'-amino-2'-desoxy-D-glucanopyranosyl)-a(1,6)-chiro-inosit;
RGL1022 O-(D-Galactopyranosyl)-α(1,4)-O-(2'-amino-2'-desoxy-D-glucanopyranosyl)-α(1,6)-chiro-inosit-1-phosphat;
Verbindung 25 O-α-D-Mannopyranosyl-(1,2)-O-α-D-mannopyranosyl-(1,6)-O-α-D-mannopyranosyl-(1,4)-O-2-ammonio-2-desoxy-α-D-glucopyranosyl-(1,6)-D-chiro-inosit-1-phosphat; oder
Verbindung 19 O-β-D-Galactopyranosyl-(1,4)-2-ammonio-2-desoxy-α-D-galactopyranosyl-(1,6)-D-chiro-inosit-1-phosphat;
oder ein Salz, ein Koordinationskomplex mit Metallionen, ein Ester, eine freie Säure oder Base, ein Hydrat davon oder ein Glykolipidderivat, in dem eine von einem Phospholipase-Enzym spaltbare Lipidgruppierung ein Substituent des Zuckerrests oder des Cyclits ist.

10. Zusammensetzung, die eine Verbindung nach einem der vorangegangenen Ansprüche in Kombination mit einem pharmazeutisch annehmbaren Träger umfasst.

11. Verbindung nach einem der Ansprüche 1 bis 9 zur Verwendung in der Therapie.

12. Verwendung einer Verbindung nach einem der Ansprüche 1 bis 9 zur Herstellung eines Medikaments zur Behandlung eines Leidens, das durch Verabreichung eines zweiten Inositphosphoglycan- (IPG-) Messengers oder eines IPG-Antagonisten gelindert wird.

## Revendications

1. Composé représenté par la formule générale :
Y-X-1,6-chiro-inositol
dans laquelle :
X représente un radical de sucre ;
Y représente un à trois radicaux de sucre ;
le résidu de sucre est non substitué ou substitué par un à quatre groupes, et le cyclitol est non substitué ou est en outre substitué par un à quatre groupes, le groupe ou les groupes étant indépendamment choisis parmi :
(a) un groupe phosphoryle, qui est un phosphate -O-P-(O)(OH)₂ ; un thiophosphate -O-P(S)(OH)₂ ; des esters de phosphate -O-P(O)(OR)₂ ; des esters de thiophosphate -O-P(S) (OR)₂ ; un phosphonate -O-P (O) OHR ; un thiophosphonate -O-P(S)OHR ; un phosphonate substitué -O-P(O)OR₁R₂ ; un thiophosphonate substitué -O-P (S) OR₁R₂ -O-P (S) (OH) (SH) ; ou un phosphate cyclique ;
(b) un composé contenant du phosphore qui est un phosphoramidite -O-P(OR)-NR₁R₂ ou un phosphoramidate -O-P(O)(OR)-NR₁R₂ ;
(c) un groupe soufré qui est -O-S(O)(OH), -SH, -SR, -S(-O)-R, -S(O)₂R,RO-S(O)₂, -O-SO₂NH₂, -O-SO₂R₁R₂ ou un sulfamide -NHSO₂NH₂;
(d) un groupe amino qui est -NHR, -NR₁R₂, -NHAc, -NHCOR, -NH-O-COR, -NHSO₃, - NHSO₂R ou -N(SO₂R)₂, et/ou un groupe amidino qui est -NH-C(=NH)NH₂ et/ou un groupe uréido qui est -NH-CO-NR₁R₂ ou un groupe thiouréido qui est -NH-C(S)-NH₂ ;
(e) un groupe hydroxy ou un groupe hydroxy substitué qui est -OR₃, où R₃ est un groupe alkyle en C_{1 à 10} non substitué ou substitué, par exemple CHF₂ ou CF₃, alcoxyalkyle, aryloxyalkyle, cycloalkyle, alcényle (alkyle substitué), alkylène (cycloalkyle en C_{3 à 7}), -OCOR, aryle, hétéroaryle, acétal, ou bien où deux groupes hydroxyle sont joints sous forme de cétal ;
(f) un substituant halogène, qui est le fluor ou le chlore ;
(g) un atome d'hydrogène, pour former un désoxyose;
dans lesquels R, R₁ et R₂ sont indépendamment un atome d'hydrogène ou un groupe alkyle ou aryle en C₁ à 10 non substitué ou substitué ;
ou un sel, un complexe de coordination avec des ions de métaux, un ester, un acide ou une base libre, un hydrate de celui-ci, ou un dérivé glycolipide dans lequel une fraction lipide pouvant être clivée par une enzyme phospholypase est un substituant d'un résidu de sucre ou du cyclitol.

2. Composé selon la revendication 1, dans lequel le résidu de sucre et le chiro-inositol sont liés en α.

3. Composé selon la revendication 1, dans lequel le résidu de sucre et le chiro-inositol sont liés en β.

4. Composé selon l'une quelconque des revendications précédentes, dans lequel le résidu de sucre est un hexose ou un pentose, ou des formes substituées de ceux-ci.

5. Composé selon la revendication 4, dans lequel le résidu de sucre est un hexose choisi dans le groupe constitué par le glucose, le galactose ou le mannose.

6. Composé selon la revendication 4, dans lequel le résidu de sucre est une hexosamine.

7. Composé selon la revendication 6, dans lequel l'hexosamine est la galactosamine ou la glucosamine.

8. Composé selon l'une quelconque des revendications précédentes, dans lequel le chiro-inositol est un énantiomère D ou L.

9. Composé selon l'une quelconque des revendications précédentes, qui est :
RGL1021 *O*-(D-galactopyranosyl)-α(1,4)-*O*-(2'-amino-2'-désoxy-D-glucanopyranosyl)-α(1,6)-*chiro-*inositol ;
RGL1022 *O*-(D-galactopyranosyl)-α(1,4)-*O*-(2'-amino-2'-désoxy-D-glucanopyranosyl)-α(1,6)-*chiro*-inositol-1-phosphate ;
Composé 25 *O*-α-D-mannopyranosyl-(1,2)-*O*-α-D-mannopyranosyl-(1,6)-*O*-α-D-mannopyranosyl-(1,4)-*O*-2 ammonio-2-désoxy-α-D-glucopyranosyl-(1,6)-D-*chiro-*inositol-1-phosphate ; et
Composé 19 *O*-β-D-galactopyranosyl-(1,4)-2-ammonio-2-désoxy-α-D-galactopyranosyl-(1,6)-D-*chiro*-inositol-1-phosphate ;
ou un sel, un complexe de coordination avec des ions de métaux, un ester, un acide ou une base libre, un hydrate de celui-ci, ou un dérivé glycolipide dans lequel une fraction lipide pouvant être clivée par une enzyme phospholipase est un substituant d'un résidu de sucre ou du cyclitol.

10. Composition comprenant un composé selon l'une quelconque des revendications précédentes, en combinaison avec un support pharmaceutiquement acceptable.

11. Composé selon l'une quelconque des revendications 1 à 9, à utiliser en thérapie.

12. Utilisation d'un composé selon l'une quelconque des revendications 1 à 9, pour la préparation d'un médicament destiné au traitement d'une affection améliorée par l'administration d'un second messager d'inositol phosphoglycane (IPG) ou d'un antagoniste d'IPG.
